# EUROPEAN PATENT SPECIFICATION

(11) **EP 1 359 153 B1**
(45) Date of publication and mention of the grant of the patent: **21.06.2006**
(21) Application number: 03015238.3
(22) Date of filing: 14.04.2000
(51) Int. Cl.: C07D 501/00, A61K 31/545, A61P 31/00

(54) **Beta-lactamase inhibiting compounds**
Beta-Lactamase hemmende Verbindungen
Composés inhibiteurs de béta-lactamase

(30) Priority: 15.04.1999 US 129482 P
(43) Date of publication of application: 05.11.2003
(62) Divisional of application: 00922157.3
(73) Proprietor: Research Corporation Technologies, Inc, Tucson, Arizona 85711-3335 (US)
(72) Inventor: Buynak, John D., Dallas, Texas 75238 (US); Nidamarthy, Sirishkumar D., Pottstown PA 19464 (US); Adam, Greg C., San Diego, CA 92130 (US); Rao, Srinivasa A., Lowell MA 01851 (US); Doppalapudi, Venkata Ramana, Apt. 202, Dallas, Texas 75206 (US)
(74) Representative: Hay, Martin Alexander

(56) References cited:
- WO-A-96/17849
- US-A- 5 760 027
- BUYNAK J D ET AL: "ALKYLIDENECEPHALOSPORIN ESTERS AS INHIBITORS OF HUMAN LEUKOCYTE ELASTASE" JOURNAL OF MEDICINAL CHEMISTRY,US,AMERICAN CHEMICAL SOCIETY. WASHINGTON, vol. 40, no. 21, 10 October 1997 (1997-10-10), pages 3423-3433, XP002063391 ISSN: 0022-2623

## Description

### Background of the Invention

The most important mechanism of microbial resistance to β-lactam antibiotics is the bacterial production of β-lactamases, enzymes which hydrolytically destroy β-lactam antibiotics, such as penicillins and cephalosporins. This type of resistance can be transferred horizontally by plasmids that are capable of rapidly spreading the resistance, not only to other members of the same strain, but even to other species. Due to such rapid gene transfer, a patient can become infected with different organisms, each possessing the same β-lactamase.

β-lactamase enzymes have been organized into four molecular classes: A, B, C, and D based on amino acid sequence. Class A, which includes RTEM and the β-lactamase of Staphylococcus aureus, class C, which includes the lactamase derived from P-99 Enterobacter cloacae, and class D are serine hydrolases. Class A enzymes have a molecular weight of about 29 kDa and preferentially hydrolyze penicillins. The class B lactamases are metalloenzymes and have a broader substrate profile than the proteins in the other classes. Class C enzymes include the chromosomal cephalosporinases of Gram-negative bacteria and have molecular weights of approximately 39 kDa. The recently recognized class D enzymes exhibit a unique substrate profile which differs significantly from both class A and class C.

The class C cephalosporinases, in particular, are responsible for the resistance of gram negative bacteria to a variety of both traditional and newly designed antibiotics. The Enterobacter species, which possesses a class C enzyme, is now the third greatest cause of nosocomial infections in the United States. This class of enzymes often has poor affinities for inhibitors of the class A enzymes, such as clavulanic acid, a commonly prescribed inhibitor, and to common in vitro inactivators, such as 6-β-iodopenicillanate.

One strategy for overcoming this rapidly evolving bacterial resistance is the synthesis and administration of β-lactamase inhibitors. Frequently, β - lactamase inhibitors do not possess antibiotic activity themselves and are thus administered together with an antibiotic. One example of such a synergistic mixture is the product sold under the trademark AUGMENTIN (amoxicillin, clavulanate potassium), which contains the antibiotic amoxicillin and the β-lactamase inhibitor, clavulanate potassium.

There is a continued need for novel β-lactamase inhibitors, and in particular, for β-lactamase inhibitors that can be coadministered with a β-lactam antibiotic.

### Summary of the Invention

The invention -provides a compound of formula IV: 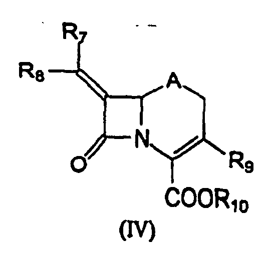 wherein:
R₇ and R₈ are each independently hydrogen, (C₁-C₁₀)alkyl, (C₂₋C₁₀)alkenyl, (C₂-C₁₀)alkynyl, (C₃-C₈)cycloakl, (C₁-C₁₀)alkoxy, (C,-C₁₀)alkanoyl, (C₁-C₁₀)alkanoyloxy. (C₁-C₁₀)alkoxycarbonyl, aryl, heterocycle, halo, cyano, nitro, -COORₑ, -C(=O)NR_{f}R_{g}, -OC(=O)NR_{f}R_{g}, NR_{f}R_{g}, or -S(O)ₙRₕ;
R₉ is cyano, -CH=NORᵢ, or a radical of the following formula 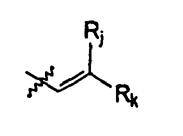 R₁₀ is hydrogen;
A is thio, sulfinyl, or sulfonyl;
each n is independently 0, 1, or 2;
each Rₑ is independently hydrogen, or (C₁-C₁₀)akyl;
each R_{f} and R₈ is independently hydrogen, (C₁-C₁₀)alkyl, (C,-C₁₀)alkoxy, phenyl, benzyl, phenethyl, or (C₁-C₁₀)akanoyl;
each Rₕ is independently (C₁-C₁₀)alkyl, phenyl, aryl(C₁-C₆)alkyl, heterocycle, or heterocycle(C₁-C₆)alkyl;
Rᵢ is hydrogen or (C₁-C₆)alkyl; and
Rⱼ and Rₖ are each independently hydrogen, halo, cyano, nitro, aryl, heterocycle, (C₂-C₆)alkenyl, -COOR_{c}, -C(=O)NR_{f}R_{g}, -OC(=O)NR_{f}R_{g}, NR_{f}R_{g}, or -S(O)ₙRₕ;
wherein any (C₁-C₁₀)alkyl, (C₂-C₁₀)alkenyl, (C₂-C₁₀)alkynyl, (C₃₋C₈)cycloalkyl, (C₁-C₁₀)alkoxy, (C₁-C₁₀)alkanoyl, (C₁-C₁₀)alkanoyloxy, or (C,-C₁₀)alkoxycarbonyl of R₇, R₈, Rⱼ and Rₖ is optionally substituted with one or more (e.g. 1, 2, 3, or 4) substituents independently selected from halo, hydroxy, C₆)alkynyl, (C₁- C₆)alkoxy, (C₁-C₆)alkanoyl, (C₁-C₆)alkanoyloxy, aryl(C₁₋C₆)alkanoyloxy, halo(C₁-C₆)alkanoyloxy, heterocycle(C₁-C₆)alkanoyloxy, aryloxy, (heterocycle)oxy, (C₃-C₈)cycloalkyl, -COOR_{c}, -C(=O)NR_{f}R_{g},-OC(=O)NR_{f}R_{g}, NRₕR_{f}, or -S(O)ₙRₖ; and
wherein any aryl is optionally substituted with one or more (e.g. 1, 2, 3, or 4) substituents independently selected from halo, hydroxy, cyano, trifluoromethyl, nitro, trifluoromethoxy, (C₁-C₆)alkyl, (C₁-C₆)alkanoyl, (C,-C₆)alkanoyloxy, (C₁-C₆)alkoxycarbonyl,-COOR_{c}, -C(=O)NR_{f}R_{g}, -OC(=O)NR_{f}R_{g}, NRₕRᵢ, or -S(O)ₙRₖ;
or a pharmaceutically acceptable salt thereof.

The invention also provides a pharmaceutical composition comprising a compound of formula IV, or a pharmaceutically acceptable salt thereof, in combination with a pharmaceutically acceptable diluent or carrier, as well as such a pharmaceutical composition that further comprises a β-lactam antibiotic.

The invention also provides a method comprising inhibiting a β-lactamase by contacting (*in vitro*) the β-lactamase with an effective amount of a compound of formula IV; or a pharmaceutically acceptable salt thereof.

The invention also provides a compound of formula IV for use in medical therapy (preferably for use in inhibiting a β-lactamase in a mammal, or for treating a β-lactam resistant bacterial infection in a mammal), as well as the use of a compound of formula IV for the manufacture of a medicament useful for inhibiting a β-lactamase in a human.

The invention also provides processes and intermediates disclosed herein that are useful for preparing β-lactamase inhibitors of formula I or IV.

Compounds of formula IV are useful as β-lactamase inhibitors for therapeutic applications. They are also useful as pharmacological tools for *in vitro* or *in vivo* studies to investigate the mechanisms of antibiotic resistance, to help identify other therapeutic antibiotic agents or β-lactamase inhibitors, to identify which β-lactamases are being expressed by a given microorganism, or to selectively inhibit one or more β-lactamases in a microorganism.

### Brief DeScription of the Figure

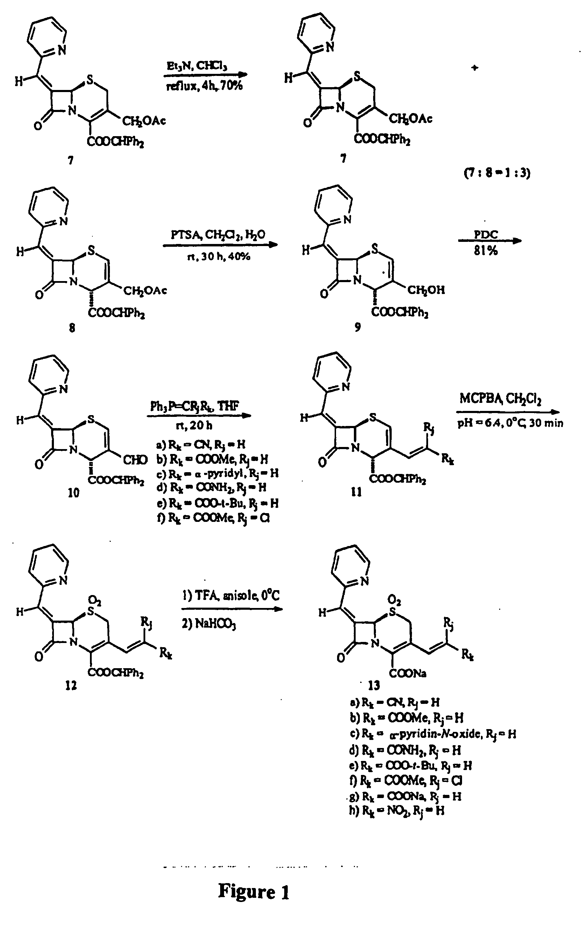

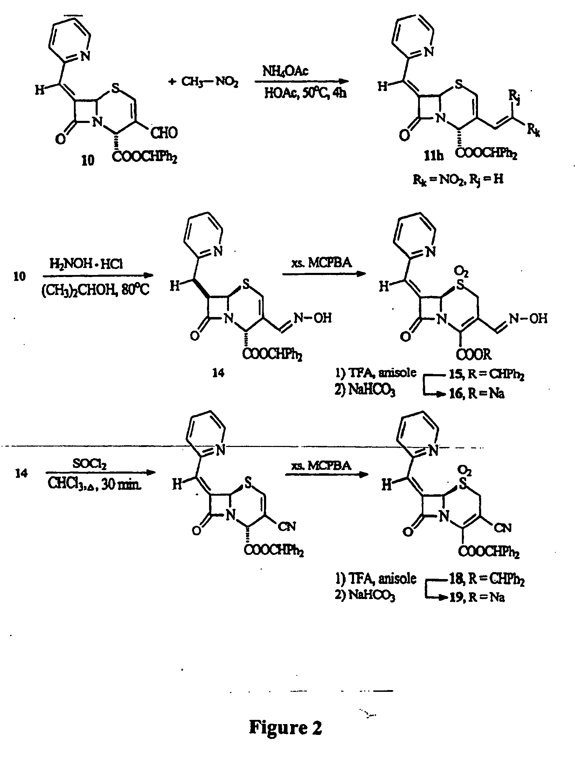

Figures 1 and 2 Illustrate the synthesis of compounds of formula IV

### Detailled Description of the Invention

The following definitions are used, unless otherwise described: halo is fluoro, chloro, bromo, or iodo. Alkyl, alkoxy, alkenyl, etc. denote both straight and branched groups. Aryl denotes a phenyl radical or an ortho-fused bicyclic carbocyclic radical having about nine to ten ring atoms in which at least one ring is aromatic. Heterocycle denotes a 6-10 membered unsaturated or saturated mono- bi- or tri-cyclic ring system comprising carbon and 1, 2, 3, or 4 heteroatoms selected from the group consisting of non-peroxide oxygen, sulfur, and N(X) wherein each X is absent or is H, O, (C₁-C₄)alkyl, phenyl or benzyl. The term "heterocycle" includes "heteroaryl," which denotes a radical attached via a ring carbon of a monocyclic aromatic ring containing five or six ring atoms consisting of carbon and one to four heteroatoms each selected from the group consisting of non-peroxide oxygen, sulfur, and N(X) wherein each X is absent or is H, O, (C₁-C₄)alkyl, phenyl or benzyl, as well as a radical of an ortho-fused bicyclic heterocycle of about eight to ten ring atoms derived therefrom, particularly a benz-derivative or one derived by fusing a propylene, trimethylene, or tetramethylene diradical thereto. The term "enhancing" the activity of a β-lactam antibiotic means improving or increasing the antibiotic activity of the compared in a statistically measurable and significant manner with respect to the activity demonstrated by the compound in the absence of a compound of the invention.

It will be appreciated by those skilled in the art that compounds of the invention having a chiral center may exist in and be isolated in optically active and racemic forms. Some compounds may exhibit polymorphism. It is to be understood that the present invention encompasses any racemic, optically-active, polymorphic, or stereoisomeric form, or mixtures thereof, of a compound of the invention, which possess the useful properties described herein, it being well known in the art how to prepare optically active forms (for example, by resolution of the racemic form by recrystallization techniques, by synthesis from optically-active starting materials, by chiral synthesis, or by chromatographic separation using a chiral stationary phase) and how to determine β-lactamase inhibitory activity using the standard tests described herein, or using other similar tests which are well known in the art.

Specific values listed below for radicals, substituents and ranges, are for illustration only; they do not exclude other defined values or other values within defined ranges for the radicals and substituents.

Specifically, (C₁-C₆)alkyl can be methyl, ethyl, propyl, isopropyl, butyl, iso-butyl, sec-butyl, pentyl, 3-pentyl, or hexyl; (C₁-C₁₀)alkyl can be methyl, ethyl, propyl, isopropyl, butyl, iso-butyl, sec-butyl, pentyl, 3-pentyl, hexyl, heptyl, octyl, nonyl, or decyl; (C₃-C₈)cycloalkyl can be cyclopropyl, cyclobutyl, cyclopentyl, cyclohexyl, cycloheptyl, or cyclooctyl; (C₁-C₁₀)alkoxy can be methoxy, ethoxy, propoxy, isopropoxy, butoxy, iso-butoxy, sec-butoxy, pentoxy, 3-pentoxy, hexyloxy, heptyloxy, octyloxy, nonyloxy or decyloxy; (C,-C₆)alkoxy can be methoxy, ethoxy, propoxy, isopropoxy, butoxy, iso-butoxy, sec-butoxy, pentoxy, 3-pentoxy, or hexyloxy; (C₂-C₁₀)alkenyl can be vinyl, 1-propenyl, 2-propenyl, 1- butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, 5-hexenyl, 1-heptenyl, 2-heptenyl, 3-heptenyl, 4-heptenyl, 5-heptenyl, 6-heptenyl, 1-octenyl, 2-octenyl, 3-octenyl, 4-octenyl, 5-octenyl, 6-octenyl, 7-octenyl, 1-nonenyl, 2-nonenyl, 3-nonenyl, 4-nonenyl, 5-nonenyl, 6-nonenyl, 7-nonenyl, 8-nonenyl, 1-decenyl, 2-decenyl, 3-decenyl, 4-decenyl, 5-decenyl, 6-decenyl, 7-decenyl, 8-decenyl, or 9-decenyl; (C₂- C₆)alkenyl can be vinyl, 1-propenyl, 2-propenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, or 5-hexenyl; (C₂₋C₁₀)alkynyl can be ethynyl, 1-propynyl, 2-propynyl, 1- butynyl, 2-butynyl, 3-butynyl, 1-pentynyl, 2-pentynyl, 3-pentynyl, 4-pentynyl, 1- hexynyl, 2-hexynyl, 3-hexynyl, 4-hexynyl, 5-hexynyl, 1-heptynyl, 2-heptynyl, 3-heptynyl, 4-heptynyl, 5-heptynyl, 6-heptynyl, 1-octynyl, 2-octynyl, 3-octynyl, 4-octynyl, 5-octynyl, 6-octynyl, 7-octynyl, 1-nonynyl, 2-nonynyl, 3-nonynyl, 4-nonynyl, 5-nonynyl, 6-nonynyl, 7-nonynyl, 8-nonynyl, 1-decynyl, 2-decynyl, 3-decynyl, 4-decynyl, 5-decynyl, 6-decynyl, 7-decynyl, 8-decynyl, or 9-decynyl; (C₂₋C₆)alkynyl can be ethynyl, 1-propynyl, 2-propynyl, 1-butynyl, 2-butynyl, 3-butynyl, 1-pentynyl, 2-pentynyl, 3-pentynyl, 4-pentynyl, 1- hexynyl, 2-hexynyl, 3-hexynyl, 4-hexynyl, or 5-hexynyl; (C₁-C₁₀)alkanoyl can be acetyl, propanoyl, butanoyl, pentanoyl, hexanoyl, heptanoyl, octanoyl, nonanoyl, or decanoyl; (C₁₋C₆)alkanoyl can be acetyl, propanoyl, butanoyl, pentanoyl, hexanoyl; (C,-C₁₀)alkoxycarbonyl can be methoxycarbonyl, ethoxycarbonyl, propoxycarbonyl, isopropoxycarbonyl, butoxycarbonyl, pentoxycarbonyl, hexyloxycarbonyl, heptyloxycarbonyl, octyloxycarbonyl, nonyloxycarbonyl or decyloxycarbonyl; (C₁-C₁₀)alkanoyloxy can be formyloxy, acetoxy, propanoyloxy, butanoyloxy, isobutanoyloxy, pentanoyloxy, hexanoyloxy, heptanoyloxy, octanoyloxy, nonanoyloxy, or decanoyloxy; (C₁- C₆)alkanoyloxy can be formyloxy, acetoxy, propanoyloxy, butanoyloxy, isobutanoyloxy, pentanoyloxy, or hexanoyloxy; halo(C₁-C₆)alkanoyloxy can be iodoacetoxy, bromoacetoxy, chloroacetoxy, fluoroacetoxy, trifluoroacetoxy, 3-chloropropanoyloxy, 3-fluoropropanoyloxy, perfluoropropanoyloxy, or 3,3,3-trifluoropropanoyloxy; aryl can be phenyl, indenyl, or naphthyl; heterocycle can be triazolyl, triazinyl, oxazoyl, isoxazolyl, oxazolidinoyl, isoxazolidinoyl, thiazolyl, isothiazoyl, pyrazolyl, imidazolyl, pyrrolyl, pyrazinyl, pyridinyl, morpholinyl, quinolinyl, isoquinolinyl, indolyl, pyrimidinyl, piperidinyl, pynolidinyl, morpholinyl, thiomorpholinyl, or piperazinyl, and heteroaryl can be furyl, imidazolyl, triazolyl, triazinyl, oxazoyl, isoxazoyl, thiazolyl, isothiazoyl, pyrazolyl, pyrrolyl, pyrazinyl, tetrazolyl, 1-methyl-1H-tetrazol-5-yl, pyridyl, (or its N-oxide), thienyl, pyrimidinyl (or its N-oxide), indolyl, isoquinolyl (or its N-oxide) or quinolyl (or its N-oxide).

Specifically, R₁ is aryl, heterocycle, or -COOR₂,

Specifically, R₁ is 2-pyridyl, or -COOR₂,

Specifically, R₂ is hydrogen.

Specifically, R₃ is hydrogen, carboxy, or -CH₂M wherein M is hydrogen, halo, hydroxy, (C₃-C₈)cycloalkyl, (C₁-C₆)alkoxy, aryloxy, aryl(C₁₋C₁₀)alkoxy, mercapto, (C₁-C₁₀)alkylthio, arylthio, heterocycle, (heterocycle)thio, (C₁-C₁₀)alkanoylthio, aminocarbonyloxy, or NR_{b}R_{c}. More specifically, M is hydrogen, halo, (C₁-C₁₀)alkanoyloxy, or heterocycle.

Specifically, R₃ is acetoxymethyl, phenylacetoxymethyl, (3,4-dihydroxyphenyl)acetoxymethyl, chloromethyl, formyl, or chloroacetoxymethyl.

Specifically, R₃ is hydrogen, methyl, acetoxymethyl, or 1-methyl-1*H*-tetrazol-5-ylthiomethyl.

Specifically, R₃ is vinyl, optionally substituted at the 2-position with halo, cyano, -COORₐ, trifluoromethyl, formyl, (C₃-C₈)cycloalkyl, (C₂₋C₆)alkenyl, (C₂-C₆)alkynyl, heterocycle, or NR_{b}R_{c}; Specifically, R₃ is vinyl, optionally substituted at the 2-position with cyano, -COORₐ, (C₂-C₆)alkenyl, or heteroaryl

Specifically, A is sulfonyl (-SO₂-).

Specifically, R₇ is aryl, heterocycle, or -COORₑ.

Specifically, R₇ is 2-pyridyl, or -COOR_{c}

Specifically, R₈ is hydrogen.

Specifically, Rⱼ and Rₖ are each independently hydrogen, cyano, - COOR_{c}, (C₂-C₁₀)alkenyl, or heteroaryl.

More specifically R₁ is carboxy, 2-pyridyl, *tert*-butoxycarbonyl, or methoxycarbonyl.

More specifically, R₃ is 2-cyanovinyl, 2-(methoxycarbonyl)vinyl, 2- (2-pyridyl-N-oxide)vinyl, or 1,3-butadienyl.

More specifically, R₅ and R₆ are each individually hydrogen.

More specifically, R₅ and R₆ are each individually methylthio

More specifically, Rⱼ and Rₖ are each independently hydrogen, cyano, 2-(methoxycarbonyl), 2-pyridyl-N-oxide, or vinyl.

A specific compound is a compound of formula IV wherein A is sulfonyl (-SO₂-); R₇ is 2-pyridyl, carboxy or *tert*-butoxycatbonyl; R₈ is hydrogen; and R₉ is 2-cyanovinyl, 2-(methoxycarbonyl)vinyl, 2-(2'-pyridyl-N-oxide)vinyl, or 1,3-butadienyl; or a pharmaceutically acceptable salt thereof.

A preferred compound of formula IV is a pharmaceutically acceptable salt formed from a carboxylic acid of formula IV wherein R₄ or R₁₀ is hydrogen. Most preferred is a salt wherein R₄ or R₁₀ has been replaced with a sodium or potassium ion. The term pharmaceutically acceptable salts also includes poly salts (e.g. di- or tri-salts) of a compound of formula IV, particularly a dicarboxylic acid salt of a compound of formula IV wherein R₇ is carboxy and R₁₀ is hydrogen

Processes and novel intermediates useful for preparing compounds of formula IV are provided as further embodiments of the invention and are illustrated by the following procedures in which the meanings of the generic radicals are as given above unless otherwise qualified. Additional compounds of the present invention, processes of making compounds of the present invention, and/or intermediates of compounds of the present invention are disclosed in Buynak et al., Bioorg. Med. Chem. Lett., 2000, 10 (2000) 4211 and Buynak et al., Bioorg Med Chem Lett., 2000, 10 (2000) 4215.

Pharmaceutically acceptable salts of compounds of formula IV wherein R₁₀ has been replaced with a pharmaceutically acceptable cation (e.g. a sodium or potassium ion) can conveniently be prepared from a corresponding compound of formula IV wherein R₁₀ is hydrogen, by reaction with a suitable base.

A useful intermediate for preparing a compound of formula IV, wherein R₄ or R₁₀ is hydrogen, is a corresponding compound wherein R₄ or R₁₀ has been replaced with a suitable removable carboxy protecting group. Such protecting groups are well known in the art, for example, see Greene, T.W.; Wutz, P.G.M. "Protecting Groups In Organic Synthesis" second edition, 1991, New York, John Wiley & Sons, Inc. Preferred protecting groups include (C,-C₁₀)alkyl, (C₂- C₁₀)alkenyl, (C₃-C₈)cycloalkyl, (C₂-C₁₀)alkynyl, aryl, benzyl, or benzhydryl. Thus the invention provides compounds of formula I wherein R₁, R₂, R₃, R₅, and R₆ have any of the values, specific values, or preferred values defined herein, and wherein R₄ is (C₁-C₁₀)alkyl, (C₂-C₁₀)alkenyl, (C₃₋C₈)cycloalkyl, (C₂-C₁₀)alkynyl, aryl, benzyl, or benzhydryl. The invention also provides compounds of formula IV wherein R₇, R₈, and R₉ have any of the values, specific values, or preferred values defined herein, and wherein R₁₀ is (C₁-C₁₀)alkyl, (C₂-C₁₀)alkenyl, (C₃-C₈)cycloalkyl, (C₂- C₁₀)alkynyl, aryl; benzyl, and benzhydryl.

As illustrated in Figure 1, compounds of formula IV (e.g. wherein R₇ is 2-pyridyl and R₈ is hydrogen) can be prepared from compound **7**, which is available from **2**, using procedures similar to those described by J. D. Buynak et. al., *J. Med. Chem. 38,* 1022-1034, 1995. Thus, **7** is isomerized to a 1:3 mixture of **7** and **8**, respectively. Compound **8** is separated and hydrolyzed to alcohol **9**, which is oxidized to provide aldehyde **10.** Reaction of **10** with a series of ylides provides compounds **11a** through **11f**. Compound **10** can also be condensed with nitromethane to produce nitroalkene **11h** (Figure 4), or can be reacted with hydroxylamine hydrochloride to produce oxime **14** (Figure 4), which itself can be reacted with thionyl chloride to generate nitrile **17**. Oxidation of compounds **11a-11f, 11h,** 14, and **17** yields the corresponding sulfones. Compound **12e** can also be reacted with Eschenmoser's salt to generate sulfone **20** (Figure 4). Hydrolysis of the esters **12a-12f, 12h, 15, 18,** and **20** (e.g. with trifluoroacetic acid (TFA) in anisole) yields the carboxylate salts **13a-13f, 13h, 16, 19,** and **21,** which are active as β-lactamase inhibitors. Compound **13g** was prepared by hydrolysis of compound **12e.**

A compound that is particularly useful for preparing a compound of formula IV is an aldehyde of formula V: 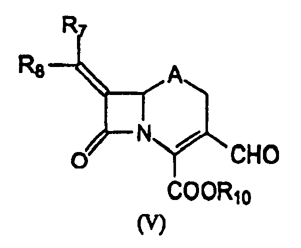 wherein R₇, R₈, R₁₀, and A have any of the values, specific values, or preferred values described herein.

A compound of formula IV wherein A is sulfonyl (-SO₂-) can be prepared by oxidation of a corresponding compound of formula IV wherein A is thio (-S-), for example, by using *meta*-chloroperbenzoic acid (mCPBA).

A compound of formula IV wherein A is sulfinyl (-SO-) can be prepared by oxidation of a corresponding compound of formula IV wherein A is thio (-S-), using one equivalent of an acceptable oxidizing agent, for example, mCPBA.

Another useful intermediate for the preparation of a compound of the invention is an ylide, for example a ylide of formula R₁R₂C=PPh₃, R₅R₆C=PPh₃, or RⱼRₖC=PPh₃. Ylides can be prepared using techniques that are well known in the art, for example techniques such as those described in Jerry March "Advanced Organic Chemistry" John Wiley & Sons, 4 ed.1992, 956-963. Suitable ylides are also disclosed in U.S. Patent Number 5,597,817, issued January 29, 1997; and U.S. Patent Number 5,629,306, issued May 13, 1997.

Compounds of formula IV wherein A is -S- or -SO- are particularly useful as intermediates for preparing the corresponding compounds of formula IV wherein A is -SO2-.

Many of the starting materials employed in the synthetic methods described above are commercially available or are reported in the scientific literature. It may be desirable to optionally use a protecting group during all or portions of the above described synthetic procedures. Such protecting groups and methods for their introduction and removal are well known in the art. See Greene,T.W.; Wutz, P.G.M. "Protecting Groups In Organic Synthesis" second edition, 1991, New York, John Wiley & Sons, Inc.

In cases where compounds are sufficiently basic or acidic to form stable nontoxic acid or base salts, administration of the compounds as salts may be appropriate. Examples of pharmaceutically acceptable salts are organic acid addition salts formed with acids which form a physiological acceptable anion, for example, tosylate, methanesulfonate, acetate, citrate, malonate, tartarate, succinate, benzoate, ascorbate, α-ketoglutarate, and α-glycerophosphate. Suitable inorganic salts may also be formed, including hydrochloride, sulfate, nitrate, bicarbonate, and carbonate salts.

Pharmaceutically acceptable salts may be obtained using standard procedures well known in the art, for example, by reacting a sufficiently basic compound such as an amine with a suitable acid affording a physiologically acceptable anion. Alkali metal (for example, sodium, potassium or lithium) or alkaline earth metal (for example, calcium) salts of carboxylic acids can also be made.

The compounds can be formulated as pharmaceutical compositions and administered to a mammalian host, such as a human patient in a variety of forms adapted to a selected route of administration, i.e., by oral, parenteral, intravenous, intramuscular, topical, or subcutaneous routes.

Thus, the present compounds may be systemically administered, e.g., orally, in combination with a pharmaceutically acceptable vehicle such as an inert diluent or an assimilable edible carrier. They may be enclosed in hard or soft shell gelatin capsules, may be compressed into tablets, or may be incorporated directly with the food of the patient's diet. For oral therapeutic administration, the active compound may be combined with one or more excipients and used in the form of ingestible tablets, buccal tablets, troches, capsules, elixirs, suspensions, syrups, wafers, and the like. Such compositions and preparations should contain at least 0.1% of active compound. The percentage of the compositions and preparations may, of course, be varied and may conveniently be between about 2 to about 60% of the weight of a given unit dosage form. The amount of active compound in such therapeutically useful compositions is such that an effective dosage level will be obtained.

The tablets, troches, pills, capsules, and the like may also contain the following: binders such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring may be added. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the active compound, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the active compound may be incorporated into sustained-release preparations and devices.

The active compound may also be administered intravenously or intraperitoneally by infusion or injection. Solutions of the active compound or its salts can be prepared in water, optionally mixed with a nontoxic surfactant. Dispersions can also be prepared in glycerol, liquid polyethylene glycols, triacetin, and-mixtures thereof and in oils. Under ordinary conditions of storage and use, these preparations contain a preservative to prevent the growth of microorganisms.

The pharmaceutical dosage forms suitable for injection or infusion can include sterile aqueous solutions or dispersions or sterile powders comprising the active ingredient which are adapted for the extemporaneous preparation of sterile injectable or infusible solutions or dispersions, optionally encapsulated in liposomes. In all cases, the ultimate dosage form should be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about by the use in the compositions of agents delaying absorption, for example, aluminum monostearate and gelatin.

Sterile injectable solutions are prepared by incorporating the active compound in the required amount in the appropriate solvent with various of the other ingredients enumerated above, as required, followed by filter sterilization. In the case of sterile powders for the preparation of sterile injectable solutions, the preferred methods of preparation are vacuum drying and the freeze drying techniques, which yield a powder of the active ingredient plus any additional desired ingredient present in the previously sterile-filtered solutions.

For topical administration, the present compounds may be applied in pure form, i.e., when they are liquids. However, it will generally be desirable to administer them to the skin as compositions or formulations, in combination with a dermatologically acceptable carrier, which may be a solid or a liquid.

Useful solid carriers include finely divided solids such as talc, clay, microcrystalline cellulose, silica, alumina and the like. Useful liquid carriers include water, alcohols or glycols or water-alcohol/glycol blends, in which the present compounds can be dissolved or dispersed at effective levels, optionally with the aid of non-toxic surfactants. Adjuvants such as fragrances and additional antimicrobial agents can be added to optimize the properties for a given use. The resultant liquid compositions can be applied from absorbent pads, used to impregnate bandages and other dressings, or sprayed onto the affected area using pump-type or aerosol sprayers.

Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user.

Examples of useful dermatological compositions which can be used to deliver the compounds of the invention to the skin are disclosed in Jacquet et al. (U.S. Pat. No. 4,608,392), Geria (U.S. Pat. No. 4,992,478), Smith et al. (U.S. Pat. No. 4,559,157) and Wortzman (U.S. Pat. No. 4,820,508).

The present compositions may also be prepared in suitable forms for absorption through the mucous membranes of the nose and throat or bronchial tissues and may conveniently take the form of powder or liquid sprays or inhalants, lozenges, throat paints, etc. For medication of the eyes or ears, the preparations may be presented as individual capsules, in liquid or semi-solid form, or may be used as drops, etc. Topical applications may be formulated in hydrophobic or hydrophilic bases as ointments, creams, lotions, paints, powders, etc.

For veterinary medicine, the composition may, for example, be formulated as an intramammary preparation in either long acting or quick-release bases.

Useful dosages of the compounds of the invention can be determined by comparing their *in vitro* activity, and *in vivo* activity in animal models. Methods for the extrapolation of effective dosages in mice, and other animals, to humans are known to the art; for example, see U.S. Pat. No. 4,938,949.

Generally, the concentration of the compound(s) of the invention in a liquid composition, such as a lotion, will be from about 0.1-25 wt-%, preferably from about 0.5-10 wt-%. The concentration in a semi-solid or solid composition such as a gel or a powder will be about 0.1-5 wt-%, preferably about 0.5-2.5 wt-%.

The compositions per unit dosage, whether liquid or solid may contain from 0.1% to 99% of active material (the present 7-vinylidene cephalosporins and optional antibiotic), the preferred range being from about 10-60%. The composition will generally contain from about 15 mg to about 1500 mg by weight of active ingredient based upon the total weight of the composition; however, in general, it is preferable to employ a dosage amount in the range of from about 250 mg to 1000 mg. In parenteral administration the unit dosage is usually the pure compound in a slightly acidified sterile water solution or in the form of a soluble powder intended for solution. Single dosages for injection, infusion or ingestion may be administered, i.e., 1-3 times daily, to yield levels of about 0.5 - 50 mg/kg, for adults.

The invention provides a pharmaceutical composition, comprising an effective amount of a compound of formula IV as described hereinabove; or a pharmaceutically acceptable salt thereof; and a pharmaceutically acceptable carrier. The invention also provides a pharmaceutical composition comprising an effective amount of a compound of formula IV as described hereinabove; or a pharmaceutically acceptable salt thereof; a β-lactam antibiotic; and a pharmaceutically acceptable carrier. The present compositions are preferably presented in a form suitable for absorption by the gastro-intestinal tract.

Any β-lactam antibiotic is suitable for use in the pharmaceutical composition of the invention. β-lactam antibiotics which are well known in the art include those disclosed by R.B. Morin and M. Gorin, M.Eds.; Academic Press, New York, 1982; vol. 1-3. Preferred β-lactam antibiotics, suitable for use in the pharmaceutical composition of the invention, include β-lactam antibiotics which are preferentially deactivated by Class A and Class C β-lactamase enzymes, for example, amoxicillin, piperacillio, ampicillin, ceftizoxime, cefotaxime, cefuroxime, cephalexin, cefaclor, cephaloridine, and ceftazidime.

The ability of a compound of the invention to function as a β-lactamase inhibitor can be demonstrated using the test described below, or using other tests which are well known in the art. Representative compounds of formula I were evaluated as inhibitors of the Class C β-lactamase of *Enterobacter cloacae* P- 99, a cephalosporinase, and TEM-1, a penicillinase, by relative IC₅₀ analysis. The IC₅₀ value represents the concentration of inhibitor required to effect a 50% loss of activity of free enzyme. The IC₅₀ value of each compound was determined in a manner similar to the following: Following a 10 minute incubation of a dilute solution of enzyme (2.56 nM) and inhibitor (<0.64 mM), a 50 mL aliquot of this incubation mixture was then further diluted into 1 mL nitrocefin solution, and the rate of hydrolysis was measured during a 1 minute period by monitoring the absorbance of nitrocefin as a function of time.

The data is presented in Table 1 below.

**TABLE 1 IC₅₀ Values (µM) for Inhibition of β-lactamase Derived from P-99 or TEM-1**

| No | P-99 | TEM-1 |
|---|---|---|
| 13a | 0.01 | 2.7 |
| 13b | 0.2 | 0.02 |
| 13c | 0.6 | 0.006 |
| 13e | 1.48 | - |
| 13f | 0.2 | 0.02 |
| 13g | 0.31 | 2.52 |
| 19 | 0.029 | 2.34 |

The present β-lactamase inhibitors of formula IV are particularly useful in the treatment of infections associated with Enterobacter, Citrobacter, and Serratia. These bacteria have the ability to attach to the epithelial cells of the bladder or kidney (causing urinary tract infections) and are resistant to multiple antibiotics including amoxicillin and ampicillin.

Other features of the invention will become apparent in the course of the following descriptions of exemplary embodiments which are given for illustration of the invention and are not intended to be limiting thereof.

### EXAMPLES

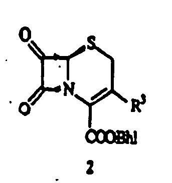

a) R³=CH₂OAc
b) R³=CH₃
c) R³=CH₂Cl

**a. Benzhydryl 7-Oxocephalosporinate (2a).** To a solution of benzhydryl 7-aminocephalosporinate (0.5 g, .15 mmol) (which can be prepared in one step from the commercially available 7-aminocephalosporanic acid according to procedures described in *Buynak et. al. J. Am. Chem Soc. 116, 10955-10965, 1994)* in ethyl acetate (5 mL) were added isopropyl nitrite (0.38 mL, 1.71 mmol, 40% solution in CH₂Cl₂) and trifluoroacetic acid (6.5 mg, 0.05 mmol) and the reaction was allowed to stir for 1 hour at room temperature. The reaction mixture was concentrated under reduced pressure and redissolved in benzene (5 mL). To this solution was added propylene oxide (6.7 g, 0.114 mol) followed by rhodium octanoate dimer (5 mg) and the reaction was stirred for 15 minutes (until evolution of nitrogen ceases). Volatiles were removed to produce the title compound **2a** (0.5 g, quantitative, 90% pure); IR (CHCl₃) 3005, 1830, 1790, 1740 cm⁻¹; ¹H NMR (CDCl₃) δ 7.39 (10 H, m), 7.05 (1 H, s), 5.32 (1 H, s), 5.07 (1 H, d, A of ABq, J = 13.9 Hz), 4.85 (1 H, d, B of ABq, J = 14.0 Hz), 3.64 (1 H, d, A of ABq, J = 18.5 Hz), 3.44 (1 H, d, B of ABq, J = 18.6 Hz), 2.05 (3 H, s); ¹³C NMR (CDCl₃) 188.4 (s), 170.3 (s), 160.1 (s), 158.7 (s), 138.8 (s), 138.6 (s) 128.4, 128.2, 128.1, 127.7, 126.9, 126.2, 80.1 (d), 65.8 (d), 62.6 (t), 27.7 (t), 20.4 (q).

**b. Benzhydryl 7-Oxo-3'-(desacetoxy)cephalosporinate (2b). To a** solution of benzhydryl 7-amino-3'-(desacetoxy)cephalosporinate (15g, 39,5 mmol) in ethyl acetate (300 mL) were added isopropyl nitrite (13.3 mL, 59.2 mmol, 40% solution in CH₂Cl₂) and trifluoroacetic acid (0.13 g, 1.18 mmol) and the reaction was allowed to stir for 1 hour at room temperature. The reaction mixture was concentrated under reduced pressure and redissolved in benzene (75 mL). To this solution was added propylene oxide (150 mL) followed by rhodium octanoate dimer (100 mg) and the reaction was stirred for 15 n-tin (until evolution of nitrogen ceases). Volatiles were removed to produce compound 2b (15 g, quantitative, >90% pure); ¹H NMR (CDCl₃) δ 7.97-7.25 (10 H, m), 6.99 (1 H, s), 5.29 (1 H, s), 3.47 (1 H, d, A of ABq, J = 17.9 Hz), 3.29 (1 H, d, B of ABq, J = 17.9 Hz), 2.18 (3 H, s).

**c. Benzhydryl (3-Chloromethyl)-7-oxocephalosporinate (2c).** To a solution of benzhydryl 7-amino-3-(chloromethyl)cephalosporinate (4 g, 9.7 mmol) in EtOAc (100 mL) was added isoamylnitrite (1.55mL, 11.6 mmol) and a catalytic amount of TFA (201) and the resulting solution was allowed to stir for 25 minutes. Volatiles were removed under reduced pressure to give a yellow solid which was identified as 7-diazo compound. The yellow solid was then dissolved in benzene (25 mL) and propylene oxide (50 mL, 1164 mmol) was added followed by rhodium octanoate (50 mg). The reaction mixture was stirred for 10 minutes and then concentrated under reduced to obtain ketone 2c as a yellow solid; ¹H NMR (CDCl₃, 400 MHZ), δ 7.45-7.35 (m, 10H), 7.0 (s, 1H), 5.3 (s, 1H), 4.34 (d, J = 13.8 Hz, 1H), 4.37 (d, J = 13.8 Hz, 1H), 3.69 (d, J = 18 Hz, 1H), 3.56 (d, J = 18 Hz,1H).

The following compounds were prepared as illustrated in Figures 1 and 2:

**Benzhydryl 3-(acetoxymethyl)-7*Z*-[(2'-pyridyl)methylidene]-3-cephem-4-carboxylate (7)** was prepared from 2 using a procedure similar to that described by J. D. Buynak *et. al. J. Med. Chem. 38,* 1022-1034, 1995.

**Benzhydryl 3-(acetoxymethyl)-7*Z*-[(2'-pyridyl)methylidene]-2-cephem-4-carboxylate (8).** A solution of 7 (7.0 g, 13.6 mmol) and Et₃N (1.58 g, 15.6 mmol in CHCl₃ was heated at reflux for 4 hours under argon. The reaction mixture was concentrated *in vacuo* and the residue was purified by flash chromatography on silica (eluent: 5 to 15% EtOAc in hexane) to provide 3.88 g (55%) **8** and 1.3 g (18.5%) 7; ¹H NMR (CDCl₃): δ 1.94 (s, 3H), 4.65, 4.56 (ABq, J = 25.5 Hz, 2H), 5.26 (s, 1H), 5.89 (s, 1H), 6.48 (s, 1H), 6.91 (s, 1H), 6.94 (s, 1H), 7.36-7.23 (m, 12H), 7.70 (t, J = 2.2 Hz, 1H), 8.60 (d, J = 4.2 Hz, 1H).

**Benzhydryl 3-(hydroxymethyl)-7*Z*-[(2'-pyridyl)methylidene]-2-cephem-4-carboxylate (9).** To a solution of **8** (1.1 g, 2.14 mmol) in CH₂Cl₂ (5 mL), was added H₂O (0.1 mL) and then a second solution of *p*-toluenesulfonic acid (0.46 g, 2.46 mmol) in CH₃OH (10 mL). The reaction was stirred for 24 to 30 hours. The solvent was removed *in vacuo.* The residue was dissolved in CH₂Cl₂ (20 mL), washed with saturated aqueous NaHCO₃, water and brine, and dried over Na₂SO₄. The solvent was removed *in vacuo* and the product purified by flash chromatography on silica (eluent: 25% EtOAc in hexane) to provide 525 mg (51%) 9; ¹H NMR (CDCl₃): δ 4.14, 4.09 (ABq, J = 16.2 Hz, 2H), 5.32 (s, 1H), 5.70 (s, 1H), 5.90 (s, 1H), 6.92 (s, 1H), 6.94 (s, 1H), 7.35-7.21 (m, 12H), 7.69 (t, J = 1.8 Hz, 1H), 8.60 (d, J = 4.5 Hz, 1H).

**Benzhydryl 3-formyl-*7Z*-[(2'-pyridyl)methylidene]-1-cephem-4-carboxylate (10).** Celite (2 g) was added to a solution of 9 (3.2 g, 6.79 mmol) in anhydrous CH₂Cl₂ at 0 °C. Pyridinium dichromate (2.93 g, 7.81 mmol) was then added in three equal portions over a 15 minute period. The reaction was allowed to stir at 0 °C for 2 hours. The reaction mixture was then filtered and the filtrate passed quickly through a small bed of silica gel and concentrated *in vacuo* to provide 2.6 g (81%) of 10; ¹H NMR (CDCl₃): δ 5.72 (s, 1H), 5.88 (s, 1H), 6.84 (s, 1H), 6.98 (s, 1H), 7.37-7.25 (m, 12H), 7.58 (s, 1H), 7.71 (t, J = 2.2 Hz, 1H), 8.60 (d, J = 3.9 Hz, 1H), 9.30 (s, 1H).

**General Procedure for the preparation of Alkenes 11a, 11b, 11d, and 11e.** A solution of aldehyde 10 (0.5 mmol) and the requisite ylide (5.0 mmol) was stirred in anhydrous THF (10 mL) at room temperature for approximately 24 hours. The solvent was removed *in vacuo* and the residue was purified by flash chromatography on silica using 5 to 20% EtOAc in hexane (in the case of compound **11d,** 2% MeOH in CH₂Cl₂ was required).

**Benzhydryl 3-[2'*E*-(cyanoethenyl)]-7*Z*-[(2"-pyridyl)methylidene]-2-cephem-4-carboxylate (11a).** Using the general procedure described above, the title compound was prepared; yield: 82%; ¹H NMR (CDCl₃): δ 5.30-5.34 (m, 2H), 5.98 (s, 1H), 6.90-6.84 (m, 2H), 6.98 (s, 1H), 7.40-7.28 (m, 13H), 7.73 (t, J = 1.6 Hz, 1H), 8.62 (d, J = 3.78 Hz, 1H).

**Benzhydryl 3-[2'*E*-(methoxycarbonylethenyl)]-7*Z*-[(2"-pyridyl)methylidene]-2-cephem-4-carboxylate (11b).** Using the general procedure described above, the title compound was prepared; yield: 96%; ¹H NMR (CDCl₃): δ 3.67 (s, 3H), 5.38 (s, 1H), 5.85 (d, J = 18.0 Hz, 1H), 5.91 (s, 1H), 6.78 (s, 1H), 6.82 (s, 1H), 6.90 (s, 1H), 7.10-7.28 (m, 13 H), 7.60-7.64 (m, 1H), 8.55 (d, J = 4.0 Hz, 1H).

**Benzhydryl 3-[2'*E*-(2"-pyridyl)ethenyl-7*Z*-[(2"'-pyridyl)methylidene]-2-cephem-4-carboxylate (11c).** To a slurry of triphenyl(2-pyridylmethyl)phosphonium chloride (0.39 g, 0.75 mmol) in anhydrous THF (20 mL) was added NaNH₂ (29 mg, 0.75 mmol) under argon and the reaction allowed to stir at room temperature for 2 hours. The reaction mixture allowed to settle for an additional 2 hours. The clear upper layer was carefully cannulated under argon into another flask containing a solution of the aldehyde 10 (235 mg, 0.5 mmol) in anhydrous CH₂Cl₂ (10 mL) at -78 °C. The reaction was stirred for 1 hour at -78 °C, and poured into a separatory funnel containing sat. aq. NH₄Cl. The organic layer was collected, dried on Na₂SO₄, and concentrated *in vacuo.* The residue was purified by flash chromatography on silica gel using 10 to 20% EtOAc in hexane to provide 0.21 g (78%) 11c; ¹H NMR (CDCl₃): δ 5.29 (s, 1H), 5.62 (s, 1H), 5.99 (s, 1H), 6,64 (d, J = 17,4 Hz, 1H), 6.66 (s, 1H), 6.92 (s, 1H), 6.95 (s, 1H), 7.21-7.33 (m, 14H), 7.62 - 7.63 (m, 1H), 7.69 (t, J = 2.8 Hz, 1H), 8.54 (d, J = 4.52 Hz, 1H), 8.60 (d, J = 3.84 Hz, 1H).

**Benzhydryl 3-[prop-1'*E*-ene-3'-amide)]-7*Z*-[(2"-pyridyl)methylidene]-2-cephem-4-carboxylate (11d).** Using a procedure similar to that described by S. Trippet, and D. M. Walker, *J. Chem. Soc.* 3874, 1959, the requisite ylide (carbamoylmethylenetriphenyl-phosphorane) was obtained as follows: A solution of carbamoylmethyltriphenyl-phosphonium chloride (5 g, 14.0 mmol), (itself obtained from chloroacetamide and triphenylphosphine in refluxing nitromethane) in H₂O (75 mL) was cooled to 0 °C and a cold (0 - 5 °C) solution of NaOH (0.56 g in 5 mL H₂O) was added in one portion. The resultant mixture was immediately filtered and washed with cold water (75 mL). The collected precipitate was dried under high vacuum to provide carbamoylmethylenetriphenylphosphorane. This ylide was reacted with aldehyde 10 according to the general procedure above to give the title compound; yield: 78%; ¹HNMR (CDCl₃): δ 5.41 (s, 1H), 5,70 (d, J = 15.5 Hz, 1H), 5.95 (s, 1H), 6.84 (s,1H), 6.87 (s, 1H), 6.96 (s, 1H), 7.23-7.36 (m, 13H), 7.69-7.72 (m, 2H), 8.60 (d, J = 4.12 Hz, 1H).

**Benzhydryl-3-[2'*E*-(*tert*-butoxycarbonyl)ethynyl]-7*Z*-[(2"-pyridyl)methylidenel-3-cephem4-carboxylate (11e)**. Using the general procedure described above, the title compound was prepared; ¹H NMR (CDCl₃): δ 1.48 (s, 9H), 5.44 (s, 1H), 5.92 (d, J =15.8 Hz, 1H), 5.96 (s, 1H), 6.78 (s, 1H), 6.87 (s, 1H), 6.94 (s, 1H), 7.13 (d, J = 15.8 Hz, 1H), 7.23-7.34 (m, 12H), 7.70 (t, J = 7.7 Hz, 1H), 8.60 (d, J = 4.12 Hz, 1H).

**Benzhydryl 3-[2'*Z*-chloro-2'-(methoxycarbonyl)ethenyl]-7*Z*-[(2"-pyridyl)methyldene]-2-cephem-4-carboxylate (11f).** A solution of methyl (triphenylphosphoranylidene) acetate (0.267 g, 0.79 mmol) in anhydrous THF was chilled to -20 °C and *N*-chlorosuccinimide (0.1 g, 0.79 mmol) and NaHCO₃ (0.1 g, 7.5 mmol) was added. After stirring for 30 minutes at -20 °C, aldehyde 10 (75 mg, 0.15 mmol) was added. The reaction was allowed to come to room temperature and was stirred for 36 hours. The solvent was removed *in vacuo* and the residue was purified by flash chromatography on silica to provide 73 mg (82%) 11f; ¹H NMR (CDCl₃): δ 3.72 (s, 3H), 5.44 (s, 1H), 5.96 (s, 1H), 6.84 (s, 1H), 6.87 (s, 1H), 6.95 (s, 1H), 7.27-7.69 (m, 13H), 7.69 (t, J = 7.34 Hz, 1H), 8.60 (d, J = 3.58, 1H).

**Benzhydryl 3-[2*'E*-nitroethenyl]-7*Z*-[(2"-pyridyl)methylidene]-2-cephem-4-carboxylate (11h).** A solution of aldehyde 10 (50 mg, 0.107 mmol), AcOH (63 mg, 1.07 mmol), and NH₄OAc (41 mg, 0.533 mmol) in CH₃NO₂, (2 mL) was heated at 50 °C for 2 hours. The reaction was monitored by tlc, and if it was not complete, an additional quantity of NH₄OAc (20 mg, 0.26 mmol) was added and stirring was continued for an additional 1 hour. Volatiles were removed *in vacuo* and the residue was redissolved in CH₂Cl₂ (20 mL). The resultant solution was washed with water, brine, and dried over Na₂SO₄. Concentration *in vacuo* and purification by flash chromatography on silica gel provided 48 mg (92%) 11h as a yellow solid; ¹H NMR (CDCl₃): δ 5.33 (s, 1H), 6.02 (s, 1H), 6.91 (s, 1H), 7.00 (s, 1H), 7.13 (s, 1H), 7.23 (d, J = 13.8 Hz, 1H), 7.27-7.36 (m, 12H), 7.50 (d, J = 13.8 Hz, 1H), 7.73 (t, J = 7.24 Hz, 1H), 8.62 (d, J = 4.22 Hz, 1H).

**Benzhydryl 3-(*E*-oximinomethyl)-7*Z*-[(2'-pyridyl)methylidene]-2-cephem-4-carboxylate (14).** A solution of aldehyde 10 (0.1 g, 0.21 mmol) and hydroxylamine hydrochloride (0.03 g, 0,42 mmol) in anhydrous isopropanol (4 mL) was heated at 80 °C for 3 hours. The solvent was then removed in vacuo and the residue dissolved in CH₂Cl₂ (20 mL). This solution was washed with 10% NaHCO₃, water, and brine and dried over Na₂O₄. Removal of the volatiles provided the oxime 14 (92 mg, 90%); ¹H NMR (CDCl₃): δ 5.61 (s, 1H), 5.78 (s, 1H), 6.64 (s, 1H), 6.88 (s, 1H), 6.95 (s, 1H), 7.22-7.38 (m, 12H), 7.65-7.68 (m, 2H), 8.60 (d, J = 4.38 Hz, 1H).

**Benzhydryl 3-cyano-7*Z*-[(2'-pyridyl)methylidene]-2-cephem-4-carboxylate (17).** To a solution of oxime 14 (0.1 g, 0.2 mmol) in anhydrous CHCl₃ (5 mL) was added SOCl₂ (25 mg, 0.2 mmol) by syringe and the reaction mixture was heated to reflux for 30 minutes. The solution was cooled, washed with 5% aq NaHCO₃, water, and brine, and dried over Na₂SO₄. Purification by flash chromatography on silica gel provided the nitrile 17 (78 mg, 82%); ¹H NMR (CDCl₃): δ 5.30 (s, 1H), 5.48 (s, 1H), 5.89 (s, 1H), 6.92 (s, 1H), 6.99 (s. 1H), 7.21-7.47 (m, 12H), 7.71 (t, J = 7.41 Hz, 1H), 8.57 (d, J = 4.4 Hz, 1H).

**General Procedure for Formation of Sulfones 12a, 12b, 12c, 12d, 12e, 12f, 12h, 15, and 18.** To a biphasic mixture of CH₂Cl₂ (20 mL) and buffer (pH = 6.4, phosphate buffer, Aldrich Chemical Co., 10 mL), was added the requisite sulfide prepared above, (0.2 mmol) and MCPBA (1.0 mmol) at room temperature. The reaction was stirred for 30 minutes. The organic layer was separated and washed with saturated aqueous NaHSO₃, saturated aqueous NaHCO₃, water, and brine. Removal of the solvent *in vacuo* provided the sulfone 12 (quantitative yield).

**Benzhydryl 3-[2'*E*-(cyanoethenyl)]-1,1-dioxo-7*Z*-[(2"-pyridyl)methylidene]-3-cephem-carboxylate (12a).** Using the general procedure described above, the title compound was prepared; ¹H NMR (CDCl₃): δ 3.81 (d, J = 17 Hz, 1H), 4.04 (d, J = 17 Hz, 1H), 5.34 (d, J = 16.5 Hz, 1H), 6.05 (s, 1H), 7.12 (s, 1H), 7.35-7.55 (m, 14H), 7.78 (s, 1H), 8.72 (s, 1H).

**Benzhydryl 1,1-dioxo-3-[2'*E*-(methoxycarbonylethenyl)]-7*Z*-[(2"-pyridyl)methylidene]-3-cephem-4-carborylate (12b).** Using the general procedure described above, the title compound was prepared; ¹H NMR (CDCl₃): δ 3,68 (s, 3H), 3,78 (d, J = 17.2 Hz, 1H), 4,02 (d, J = 17,2 Hz, 1H), 5.86-5.92 (m, 2H), 7.01 (s, 1H), 7.25-7.36 (m, 12H), 7.44 (d, 1H, J = 4.22 H, 7.66-7.70 (m, 1H), 7.87 (d, J = 15.9 Hz, 1H), 8.63 (d, J = 4.27 Hz, 1H).

**Benzhydryl 1,1-dioxo-3-{2'*E*-[2"-(pyridin-*N*-oxide)ethenyl]}-7*Z-*[(2"'-pyridyl)methylidene]-3-cephem-4-carboxylate (12c).** Using the general procedure described above, the title compound was prepared; ¹H NMR (CDCl₃): δ 4.12 (d, J = 17.1 Hz, 1H), 4.35 (d, J = 17.1 Hz, 1H), 5.27 (s, 1H), 7.03 (s, 1H), 7.26-7.69 (m, 18H), 8,17 (s, 1H), 8.66 (s, 1H).

**Benzhydryl1,1-dioxo-3-[prop-1'*E*-ene-3'-amide)]-7*Z*-[(2"-pyridyl)methylidene]-3-cephem-4-carboxylate (12d).** Using the general procedure described above, the title compound was prepared; ¹H NMR (CDCl₃): δ 3.87 (d, J = 17.1 Hz, 1H), 4.07 (d, J = 17.1 Hz, 1H), 5.44 (brs, 2H), 5.94-5.98 (m, 2H), 7.05 (s, 1H), 7.25-7.68 (m, 10H), 7.51-7.56 (m, 2H), 7.64-7.74 (m, 2H), 7.80 (d, J = 15.8 Hz, 1H), 8.68 (d, J = 4.32 Hz. 1H).

**Benzhydryl 1,1-dioxo-3-[2'*Z*-chloro-2'-(methoxycarbonyl)ethenyl]-7*Z*-[(2"-pyridyl)methylidene]-3-cephem-4-carboxylate (12f).** Using the general procedure described above, the title compound was prepared; ¹H NMR (CDCl₃): δ 3.73 (s, 3H), 3.87 (d, J =17.2 Hz, 1H), 4.02 (d, J = 17.2 Hz, 1H), 5.91 (s, 1H), 5.95 (s, 1H), 5.98 (s, 1H), 7.06 (s, 1H), 7.29-7.39 (m, 12H), 7.89-7.93 (m, 1H), 8.66 (d, J = 4.24 Hz, 1H).

**Benzhydryl 1,1-dioxo-3-[2'*E*-nitroethenyl]-7*Z*-[(2"-pyridyl)methylidene]-3-cephem-4-carboxylate (12h).** Using the general procedure described above, the title compound was prepared; ¹H NMR (CDCl₃): δ 3.94 (d, J = 18.1 Hz, 1H), 4.12 (d, J = 18.1 Hz, 1H), 5.98 (s, 1H), 6.96 (s, 1H), 7.04 (d, J = 14.6 Hz, 1H), 7.12 (s, 1H), 7.26-7.38 (m, 12H), 7.54 (d, J = 14.6 Hz. 1H), 7.74 (t, J = 7.86 Hz, 1H), 8.61 (d, J = 4.22 Hz, 1H).

**Benzhydryl 1,1-dioxo-3-(*E*-oxominomethyl)-7*Z*-[(2'-pyridyl)methylidene]-3-cephem4-carboxylate (15).** Using the general procedure described above, the title compound was prepared; ¹H NMR (CDCl₃): δ 4.08 (d, J = 17.4 Hz, 1H), 4.30 (d, J=17.4 Hz, 1H), 5.88(s, 1H), 6.99(s, 1H), 7.12 - 7.24 (m, 13H), 7.42 - 7.52 (m, 2H), 8.62 (d, J = 3.9 Hz, 1H).

**Benzhydryl 1,1-dioxo-3-cyano-7*Z-*[(2'-pyridyl)methylidene]-3-cephem-4-carboaylate (18).** Using the general procedure described above, the title compound was prepared; ¹H NMR (CDCl₃): δ 3.82 (d, J = 18.2 Hz, 1H), 4.12 (d, J = 18.2 Hz, 1H), 5.47 (s, 1H), 6.02 (s, 1H), 7.04 (s, 1H), 7.21-7.46 (m, 12H), 7.72 (t, J = 8.2 Hz, 1H), 8.66 (d, J = 4.52 Hz, 1H) .

**General Procedure for Conversion of Benzhydryl Esters (12a, 12b, 12c, 12d, 12f, 12h, 15, 18 to the Corresponding Sodium Carboxylates (13a, 13b, 13c, 13d, 13e, 13f, 13g,13h, 16, 19).** A solution of the benzhydryl ester (0.1 mmol) in anisole (3.0 mmol) was cooled in an ice-salt bath and TFA (12.0 mmol) was slowly added via syringe under argon. After 20 minutes, the volatiles were removed *in vacuo* and the residue was dissolved in EtOAc (5 mL). The EtOAc layer was extracted with aqueous NaHCO₃ (2 x 0.15 mmol in 4 mL H₂O). The combined NaHCO₃ layers were directly loaded on a column (high porous polymer, MCI gel, CHP20P, Mitsubishi Chemical Corp., White Plains, NY, approx. 75 to 150 mL of resin) and the product eluted with 5% EtOH in deionized (millipore) water. Yields were between 60 to 80%.

**Example 1: Sodium 3-[2'*E*-(cyanoethenyl)]-1,1-dioxo-7*Z* [(2"-pyridyl)methylidene]-3-cephem-4-carboxylate (13a);** ¹H NMR (D₂O): δ 5.55 (d, J = 16.4 Hz, 1H), 6.40 (s, 1H), (7.46, t, J = 5.12 Hz, 1H), 7.50 (d, J = 16.4 Hz, 1H), 7.59 (s, 1H), 7.68 (d, J = 7.76 Hz, 1H), 7.89 (t, J = 7.62 Hz, 1H), 8.62 (d, J = 4.54 Hz, 1H).

**Example 2: Sodium 1,1-dioxo-3-[2'*E*-(methoxycarbonylethenyl)]-7*Z-̅*[(2"-pyridyl)methylidene]-3-cephem4-carboxylate (13b);** ¹H NMR (D₂O): δ 3.72 (s, 3H), 5.70 (d, J = 22.0 Hz, 1H), 7.46-7.43 (m, 2H), 7.53 (s, 1H), 7.68-7.62 (m, 2H), 7.88 (t, J = 6.70 Hz, 1H), 8.60 (d, J = 4.1 Hz, 1H).

**Example 3: Sodium 1,1-dioxo-3-{2'*E*-[2"-(pyridin*-N*-oxide)ethenyl]}-7*Z-*[(2"'-pyridyl)methylidene]-3-cephem-4-carboxylate (13c);**¹H NMR (D₂O): δ 6.04 (s, 1H), 7.03 (d, J = 16.5 Hz, 1H), 7.39 (t, J = 6.9 Hz, 1H), 7.45 (t, J = 4.96 Hz, 1H), 7.52-7.62 (m, 4H), 7.80 (d, J = 8.1 Hz, 1H), 7.88 (t, J = 7.75 Hz, 1H), 8.23 (d, J = 6.46 Hz, 1H), 8.62 (d, J = 4.38 Hz).

**Example 4: Sodium 1,1-dioxo-3-[prop-1'*E*-ene-3'-amide)]-7*Z*-[(2"-pyridyl)methylidene]-3-cephem-4-carboxylate (13d);** ¹H NMR (D₂O): δ 6.04 (d, J = 15.6 Hz, 1H), 6.38 (s, 1H), 7.47 - 7.57 (m, 3H), 7.68 (d, J = 7.6 Hz, 1H), 7.89 (brs, 1H), 8.61 (s, 1H).

**Example 5: Sodium 1,1-Dioxo-3-[2'*E*-(*t*-butoxycarbonyletheny)]-7*Z*-[(2"-pyridyl)methylidene]-3-cephem-4-carboxylate (13e);** ¹H NMR (D₂O): δ 1.45 (s, 9H), 5.88 (d, J = 15.8 Hz, 1H), 6.39 (s, 1H), 7.46 (t, J = 5.09 Hz, 1H), 7.55-7.61 (m, 2H), 7.69 (d, J = 11.8 Hz, 1H), 7.90 (t, J = 8.11 Hz, 1H), 8.62 (d, J = 4.73 Hz, 1H).

**Example 6: Sodium 1,1-dioxo-3-[2'*Z*-chloro-2'-(methoxycarbonyl)ethenyl]-7*Z*-[(2"-pyridyl)methylidene]-3-cephem-4-carboxylate (13f);** ^{**1**}**H NMR (D**_{**2**}**O): δ** 3.81 (s, 3H), 6.0 (d, J = 15.8 Hz, 1H), 6.46 (s. 1H), 7.53 (t, J - 7.52 Hz, 1H), 7.64 (s, 1H), 7.74 - 7.71 (m, 1H), 7.95 (t, J = 7.77 Hz, 1H), 8.68 (d, J = 4.52 Hz, 1H).

**Example 7: Disodium 3-[2'*E*-carboxyethenyl]-1,1-dioxo-7*Z*-[(2"-pyridyl)methylidene]-3-cephem-4-carboxylate (13g);** Hydrolysis of compound 12e under the above general conditions gave a mixture of compound 13e and the title compound 13g, which was separated by chromatography to give the title compound;¹H NMR (D₂O): δ 5.92 (d, J = 15.8 Hz, 1H), 6.36 (s, 1H), 7.34 (d, J = 15.8 Hz, 1H), 7.45-7.48 (m, 1H), 7.55 (s, 1H), 7.69 (d, J = 7.8 Hz, 1H). 7.92 (t, J = 7.8 Hz, 1H), 8.63 (d, J = 3.6 Hz, 1H).

**Example 8: Sodium 1,1-dioxo-3-[2'*E*-nitroethenyl]-7*Z*-[(2"-pyridyl)methylidene]-3-cephem-4-carboxylate (13h);** ¹H NMR (D₂O): δ 6.02 (s, 1H), 7.11 (s, 1H), 7.16 (d, J = 15.6 Hz, 1H), 7.20 - 7.22 (m, 2H), 7.28 (d, J = 15.6 Hz, 1H), 7.78 (t, J = 6.2 Hz, 1H), 8.62 (d, J = 2.88 Hz, 1H).

**Example 9: Sodium 1,1-dioxo-3-(*E*-oximinomethyl)-7*Z*-[(2'-pyridyl)methylidene]-3-cephem-4-carboxylate(16);** ¹H NMR(D₂O): δ_6.31 (s, 1H), 7.39 (t, J = 7.84 Hz, 1H), 7.48 (d, J = 7.78 Hz, 1H), 7.71 - 7.74 (m, 2H), 8.35 (s, 1H), 8.36 (d, J = 6.34 Hz, 1H).

**Example 10: Sodium 1,1-diozo-3-cyano-7*Z*-[(2'-pyridyl)methylidene]-3-cephem-4-carboxylate (19);** ¹H NMR (D₂O): δ 6.40 (s, 1H), 7.49 - 7.52 (m, 1H), 7.61 (s, 1H), 7.69 - 7.74 (m, 1H), 7.93 - 7.91 (m, 1H), 8.63 (d, J = 4.6 Hz, 1H).

**Example 11:** The following illustrates representative pharmaceutical dosage terms, containing a compound of formula IV compound X'), for therapeutic or prophylactic use in humans.

| (i) Tablet 1 | mg/tablet |
|---|---|
| 'Compound X' | 100.0 |
| Lactose | 77.5 |
| Povidone | 15.0 |
| Croscarmellose sodium | 12.0 |
| Microcrystalline cellulose | 92.5 |
| Magnesium stearate | 3.0 |
| | 300.0 |

| (ii) Tablet 2 | mg/tablet |
|---|---|
| 'Compound X' | 20.0 |
| Microcrystalline cellulose | 410.0 |
| Starch | 50.0 |
| Sodium starch glycolate | 15.0 |
| Magnesium stearate | 5.0 |
| | 500.0 |

| (iii) Capsule | mg/capsule |
|---|---|
| 'Compound X' | 10.0 |
| Colloidal silicon dioxide | 1.5 |
| Lactose | 465.5 |
| Pregelatinized starch | 120.0 |
| Magnesium stearate | 3.0 |
| | 600.0 |

| (iv) Injection 1 (1 mg/mL) | mg/mL |
|---|---|
| Compound X' (free acid form) | 1.0 |
| Dibasic sodium phosphate | 12.0 |
| Monobasic sodium phosphate | 0.7 |
| Sodium chloride | 4.5 |
| 1.0 N Sodium hydroxide solution (pH adjustment to 7.0-7.5) | q.s. |
| Water for injection | q.s. ad 1 mL |

| (v) Injection 2 (10mg/mL) | mg/mL |
|---|---|
| 'Compound X' (free acid form) | 10.0 |
| Monobasic sodium phosphate | 0.3 |
| Dibasic sodium phosphate | 1.1 |
| Polyethylene glycol 400 | 200.0 |
| 01 N Sodium hydroxide solution (pH adjustment to 7.0-7.5) | q.s. |
| Water for injection | q.s. ad 1 mL |

| (vi) Aerosol | mg/can |
|---|---|
| 'Compound X' | 20.0 |
| Oleic acid | 10.0 |
| Trichloromonofluoromethane | 5,000.0 |
| Dichlorodifluoromethane | 10,000.0 |
| Dichlorotetrafluoroethane | 5,000.0 |

| (vii) Tablet 1 | mg/tablet |
|---|---|
| 'Compound X' | 100.0 |
| β-lactam antibiotic | 100.0 |
| Lactose | 77.5 |
| Povidone | 15.0 |
| Croscarmellose sodium | 12.0 |
| Microcrystalline cellulose | 92.5 |
| Magnesium stearate | 3.0 400.0 |

| (viii) Tablet 2 | mg/tablet |
|---|---|
| 'Compound X' | 20.0 |
| β-lactam antibiotic | 20.0 |
| Microcrystalline cellulose | 410.0 |
| Starch | 50.0 |
| Sodium starch glycolate | 15.0 |
| Magnesium stearate | 5.0 520.0 |

| (ix) Capsule | mg/capsule |
|---|---|
| 'Compound X' | 10.0 |
| β-lactam antibiotic | 10.0 |
| Colloidal silicon dioxide | 1.5 |
| Lactose | 465.5 |
| Pregelatinized starch | 120.0 |
| Magnesium stearate | 3.0 610.0 |

| (x) Injection 1 | mg/mL |
|---|---|
| 'Compound X' (free acid form) | 1:0 |
| β-lactam antibiotic | 2.0 |
| Dibasic sodium phosphate | 12.0 |
| Monobasic sodium phosphate | 0.7 |
| Sodium chloride | 4.5 |
| 1.0 N Sodium hydroxide solution (pH adjustment to 7.0-7.5) | q.s. |
| Water for injection | q.s. ad 1 mL |

| (xi) Injection 2 | mg/mL |
|---|---|
| 'Compound X' (free acid form) | 10.0 |
| β-lactam antibiotic | 5.0 |
| Monobasic sodium phosphate | 0.3 |
| Dibasic sodium phosphate | 1.1 |
| Polyethylene glycol 400 | 200.0 |
| 01 N Sodium hydroxide solution (pH adjustment to 7.0-7.5) | q.s. |
| Water for injection | q.s. ad 1 mL |

| (xii) Aerosol | mg/can |
|---|---|
| 'Compound X' | 20.0 |
| β-lactam antibiotic | 20.0 |
| Oleic acid | 10.0 |
| Trichloromonofluoromethane | 5,000.0 |
| Dichlorodifluoromethane | 10,000.0 |
| Dichlorotetrafluomethane | 5,000.0 |

The above formulations may be obtained by conventional procedures well known in the pharmaceutical art. "β-lactam antibiotic" can be any compound possessing antibiotic properties (e.g. amoxicillin, piperacillin ampicillin, ceftizoxime, cefotaxime, cefuroxime, cephalexin, cefaclor, cephaloridine, or ceftazidime). Although specific quantities of "Compound X" and "β-lactam antibiotic" are shown in the above illustrative examples, it is to be understood that the compounds can be present in any ratio provided the final formulation possesses the desired antibiotic properties.

## Claims

1. A compound of formula IV; 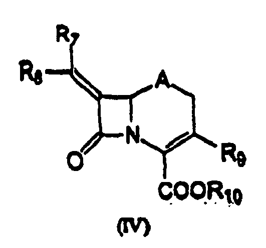 wherein :
R₇ and R₈ are each independently hydrogen, (C₁-C₁₀alkyl, (C₂₋C₁₀)alkenyl, (C₂-C₁₀)alkynyl, (C₃-C₈)cycloalkyl, (C₁-C₁₀)alkoxy, (C₁₋C₁₀)alkanoyl, (C₁-C₁₀)alkanoyloxy, (C₁-C₁₀)alkoxycarbonyl, aryl, heterocycle, halo, cyano, nitro, -COORₑ, -C(=O)NR_{f}R_{g}, -OC(=O)NR_{f}R_{g}, NR_{f}R_{g}, or -S(O)ₙRₕ;
R₉ is cyano, -CH=NORᵢ, or a radical of the following formula 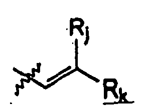
R₁₀ is hydrogen;
A is thio, sulfinyl, or sulfonyl;
each n is independently 0,1, or 2;
each R_{c} is independently hydrogen, or (C₁-C₁₀)alkyl;
each R_{f} and R_{g} is independently hydrogen, (C₁-C₁₀)alkyl, (C₁₋C₁₀)alkoxy, phenyl, benzyl, phenethyl, or (C₁-C₁₀)alkanoyl;
each Rₕ is independently (C₁-C₁₀alkyl, phenyl, aryl(C₁-C₆)alkyl, heterocycle, or heterocycle(C₁-C₆)alkyl;
R_{f} is hydrogen or (C₁-C₆)alkyl; and
R_{f} and Rₖ are each independently hydrogen, halo, cyano, nitro, aryl, heterocycle, (C₂-C₆)akenyl, -COORₑ, -C(=O)NR_{f}R_{g}, -OC(=O)NR_{f}R_{g}, NR_{f}R_{g}, or -S(O)ₚRₕ;
wherein any (C₁-C₁₀)alkyl, (C₂-C₁₀)alkenyl, (C₂-C₁₀)akynyl, (C₃₋C₈)cycloalkyl, (C₁-C₁₀)alkoxy, (C₁-C₁₀)alkanoyl, (C₁-C₁₀)alkanoyloxy, or (C₁₋C₁₀)alkoxycarbonyl of R₇, R_{g}, Rⱼ and Rₖ is optionally substituted with one or more (e.g. 1, 2, 3, or 4) substituents independently selected from halo, hydroxy, cyano, cyanato, nitro, mercapto, oxo, aryl, heterocycle, (C₂-C₆)alkenyl, (C₂₋C₆)alkynyl, (C₁-C₆)alkoxy, (C₁-C₆)alkanoyl, (C₁-C₆)alkanoyloxy, aryl(C₁₋C₆)alkanoyloxy, halo(C₁-C₆)alkanoyloxy, heterocycle(C₁-C₆)alkanoyloxy, aryloxy, (heterocycle)oxy, (C₃-C₈)cycloalkyl, -COOR_{c}, -C(=O)NR_{f}R_{g}, -OC(=O)NR_{f}R_{g}, NR_{b}R_{f}, or -S(O)ₙRₖ; and
wherein any aryl is optionally substituted with one or more (e.g. 1, 2, 3, or 4) substituents independently selected from halo, hydroxy, cyano, trifluoromethyl, nitro, trifluoromethoxy, (C₁-C₆)alkyl, (C₁-C₆)alkanoyl, (C₁₋C₆)alkanoyloxy, (C₁-C₆)alkoxycarbonyl,-COORₑ -C(O)NR_{f}R_{g}, -OC(O)NR_{f}R_{g}, NRₕRᵢ, or -S(O)ₙRₖ;
or a pharmaceutically acceptable salt thereof.

2. The compound of claim 1 wherein R₇ is aryl, heterocycle, or -COOR₈.

3. The compound of claim 1 wherein R₇ is 2-pyridyl, or -COOR₈-.

4. The compound of any one of the preceding claims wherein R₈ is hydrogen.

5. The compound of any one of the preceding claims wherein one of Rⱼ and Rₖ is hydrogen and the other is cyano, -COOR₈, (C₂-C₁₀)alkenyl, or heteroaryl.

6. The compound of any one of claims 1 - 4 wherein Rⱼ is hydrogen or halo, and Rₖ is cyano, methoxycarbonyl, aminocarbonyl, tert-butoxycarbonyl, 2-pyridyl-N-oxide, nitro, or vinyl.

7. The compound of any one of the preceding claims which is a pharmaceutically acceptable salt of a compound of formula IV wherein R₁₁ is hydrogen.

8. The compound of any one of the preceding claims wherein A is sulfonyl.

9. The compound of claim 1 wherein A is sulfonyl; R₇ is 2-pyridyl, carboxy or *tert*-butoxy carbonyl; R₈ is hydrogen; Rⱼ is hydrogen or halo; and Rₖ is cyano, methoxycarbonyl, aminocarbonyl, tert-butoxycarbonyl, 2-pyridyl-N-oxide, nitro, or vinyl.

10. A pharmaceutical composition comprising a compound of any one of claims 1 - 9, or a pharmaceutically acceptable salt thereof; and a pharmaceutically acceptable carrier.

11. The composition of claim 10 further comprising a β-lactam antibiotic.

12. The composition of claim 10 wherein the antibiotic is amoxicillin, piperacillin, ampicillin, ceftizoxime, cefotaxime, cefuroxime, cephalexin, cefaclor, cephaloridine, or ceftazidime.

13. A method comprising inhibiting a β-lactamase by contacting said β-lactamase with an effective amount of a compound of any one of claims 1 - 10 in vitro.

14. A compound of any one of claims 1 - 10 for use in medical therapy.

15. The use of a compound of any one of claims 1 - 10 for the manufacture of a medicament for reducing β-lactamase activity in a mammal.

16. A compound of formula IV 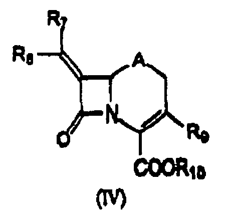 wherein:
R₇ and R₈ are each independently hydrogen, (C₁-C₁₀)alkyl, (C₂₋C₁₀)alkenyl, (C₂-C₁₀)alkynyl, (C₃-C₆)cycloalkyl, (C₁-C₁₀)alkoxy, (C₁₋C₁₀)alkanoyl, (C₁-C₁₀)alkanoyloxy, (C₁-C₁₀)alkoxycarbonyl, aryl, heterocycle, halo, cyano, nitro, -COORₑ, -C(=O)NR_{f}R_{g}, -OC(=O)NR_{f}R_{g}, NR_{f}R_{g}, or -S(O)ₙRₕ;
R₉ is cyano, -CH=NORᵢ, or a radical of the following formula 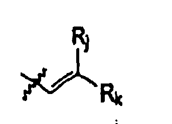
R₁₀ is (C₁-C₁₀)alkyl, (C₂-C₁₀)alkenyl, (C₃-C₈)cycloalkyl), (C₂-C₁₀)alkynyl, aryl, benzyl, and benzhydryl;
A is thio, sulfinyl, or sulfonyl;
each n is independently 0, 1, or 2;
each Rₑ is independently hydrogen, or (C₁-C₁₀)alkyl;
each R_{f} and R_{g} is independently hydrogen, (C₁-C₁₀)alkyl, (C₁-C₁₀)alkoxy, phenyl, benzyl, phenethyl, or (C₁-C₁₀)alkanoyl;
each Rₕ is independently (C₁-C₁₀)alkyl, phenyl, aryl(C₁-C₆)alkyl, heterocycle, or heterocycle(C₁-C₆)alkyl;
Rᵢ is hydrogen or (C₁-C₆)alkyl; and
Rⱼ and Rₖ are each independently hydrogen, halo, cyano, nitro, aryl, heterocycle, (C₂-C₆)alkenyl, -COORₑ, -C(=O)NR_{f}R_{g}, -OC(=O)NR_{f}R_{g}, NR_{f}R_{g}, or -S(O)ₙRₕ;
wherein any (C₁-C₁₀)alkyl, (C₂-C₁₀)alkenyl, (C₂-C₁₀)alkynyl, (C₃-C₈)cycloalkyl, (C₁-C₁₀)alkoxy, (C₁-C₁₀)alkanoyl, (C₁-C₁₀)alkanoyloxy, or (C₁-C₁₀)alkoxycarbonyl of R₇, R_{g}, Rⱼ and Rₖ is optionally substituted with one or more (e.g. 1, 2, 3, or 4) substituents independently selected from halo, hydroxy, cyano, cyanato, nitro, mercapto, oxo, aryl, heterocycle, (C₂-C₆)alkenyl), (C₂-C₆)alkynyl, (C₁-C₆)alkoxy, (C₁-C₆)alkanoyl, (C₁-C6)alkanoyloxy, aryl(C₁-C₆)alkanoyloxy, halo(C₁-C₆)alkanoyloxy, heterocycle (C₁-C₆)alkanoyloxy, aryloxy, (heterocycle)oxy, (C₃-C₈)cycloalkyl, -COOR_{g}, -C(=O)NR_{f}R_{g}, -OC(=O)NR_{f}R_{g}, NRₕRᵢ, or -S(O)ₙRₖ; and
wherein any aryl is optionally substituted with one or more (e.g. 1, 2, 3, or 4) substituents independently selected from halo, hydroxy, cyano, trifluoromethyl, nitro, trifluoromethyl, (C₁-C₆)alkyl), (C₁-C₆)alkanoyl, (C₁-C₆)alkanoyloxy, (C₁-C₆)alkoxycarbonyl, -COOR_{g}, -C(=O)NR_{f}R_{g}, -OC(=O)NR_{f}R_{g}, NRₕRᵢ, or -S(O)ₙRₖ.

## Patentansprüche

1. Verbindung der Formel IV 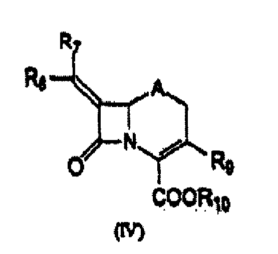 worin
R₇ und R₈ jeweils unabhängig für Wasserstoff, C₁-C₁₀ Alkyl, C₂-C₁₀ Alkenyl, C₂-C₁₀ Alkinyl, C₃-C₈ Cycloalkyl, C₁-C₁₀ Alkoxy, C₁-C₁₀ Alkanoyl, C₁-C₁₀ Alkanoyloxy, C₁-C₁₀ Alkoxycarbonyl, Aryl, einen Heterocyclus, Halogen, Cyano, Nitro, -COORₑ, -C(=O)NR_{f}R_{g}, -OC(=O)NR_{f}R_{g}, NR_{f}R_{g} oder -S(O)ₙRₕ stehen, R₉ für Cyano, -CH=NORᵢ oder einen Rest der folgenden Formel steht 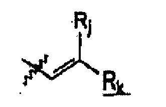 R₁₀ für Wasserstoff steht,
A für Thio, Sulfinyl oder Sulfonyl steht,
n jeweils unabhängig für 0, 1 oder 2 steht,
Rₑ jeweils unabhängig für Wasserstoff oder C₁-C₁₀ Alkyl steht,
R_{f} und R_{g} jeweils unabhängig für Wasserstoff, C₁-C₁₀ Alkyl, C₁-C₁₀ Alkoxy, Phenyl, Benzyl, Phenethyl oder C₁-C₁₀ Alkanoyl stehen,
Rₕ jeweils unabhängig für C₁-C₁₀ Alkyl, Phenyl, Aryl-C₁-C₆-alkyl, einen Heterocyclus oder Heterocyclus-C₁-C₆-alkyl steht,
Rᵢ für Wasserstoff oder C₁-C₆ Alkyl steht, und
Rⱼ und Rₖ jeweils unabhängig für Wasserstoff, Halogen, Cyano, Nitro, Aryl, einen Heterocyclus, C₂-C₆ Alkenyl, -COORₑ, -C(=O)NR_{f}R_{g}, -OC(=O)NR_{f}R_{g}, NR_{f}R_{g} oder -S(O)ₙRₙ stehen,
worin jeweils C₁-C₁₀ Alkyl, C₂-C₁₀ Alkenyl, C₂-C₁₀ Alkinyl, C₃-C₈ Cycloalkyl, C₁-C₁₀ Alkoxy, C₁-C₁₀ Alkanoyl, C₁-C₁₀ Alkanoyloxy oder C₁-C₁₀ Alkoxycarbonyl von R₇, R₈, Rⱼ und Rₖ optional mit einem oder mehreren (beispielsweise 1, 2, 3 oder 4) Substituenten substituiert sind, die unabhängig ausgewählt sind aus Halogen, Hydroxy, Cyano, Cyanato, Nitro, Mercapto, Oxo, Aryl, einen Heterocyclus, C₂-C₆ Alkenyl, C₂-C₆ Alkinyl, C₁-C₆ Alkoxy, C₁-C₆ Alkanoyl, C₁-C₆ Alkanoyloxy, Aryl-C₁-C₆-alkanoyloxy, Halogen-C₁-C₆-alkanoyloxy, Heterocyclus-C₁-C₆-alkanoyloxy, Aryloxy, (Heterocyclus)oxy, C₃-C₈ Cycloalkyl, -COORₑ, -C(=O)NR_{f}R_{g}, -OC(=O)NR_{f}R_{g}, NRₕRᵢ oder -S(O)ₙRₖ und
worin jedes Aryl wahlweise mit einem oder mehreren (beispielsweise 1, 2, 3 oder 4) Substituenten substituiert ist, die unabhängig ausgewählt sind aus Halogen, Hydroxy, Cyano, Trifluormethyl, Nitro, Trifluormethoxy, C₁-C₆ Alkyl, C₁-C₆ Alkanoyl, C₁-C₆ Alkanoyloxy, C₁-C₆ Alkoxycarbonyl, -COORₑ, -C(=O)NR_{f}R_{g}, -OC(=O)NR_{f}R_{g}, NRₕRᵢ oder -S(O)ₙRₖ,
oder ein pharmazeutisch annehmbares Salz hiervon.

2. Verbindung nach Anspruch 1, worin R₇ für Aryl, einen Heterocyclus oder -COOR₈ steht.

3. Verbindung nach Anspruch 1, worin R₇ für 2-Pyridyl oder -COOR₈ steht.

4. Verbindung nach einem der vorangehenden Ansprüche, worin R₈ für Wasserstoff steht.

5. Verbindung nach einem der vorangehenden Ansprüche, worin eines von Rⱼ und Rₖ für Wasserstoff steht und das andere für Cyano, -COOR₈, C₂-C₁₀ Alkenyl oder Heteroaryl steht.

6. Verbindung nach einem der Ansprüche 1 bis 4, worin Rⱼ für Wasserstoff oder Halogen steht und Rₖ für Cyano, Methoxycarbonyl, Aminocarbonyl, tert-Butoxycarbonyl, 2-Pyridyl-N-oxid, Nitro oder Vinyl steht.

7. Verbindung nach einem der vorangehenden Ansprüche, die ein pharmazeutisch annehmbares Salz einer Verbindung der Formel IV ist, worin R₁₁ für Wasserstoff steht.

8. Verbindung nach einem der vorangehenden Ansprüche, worin A für Sulfonyl steht.

9. Verbindung nach Anspruch 1, worin A für Sulfonyl steht, R₇ für 2-Pyridyl, Carboxy oder tert-Butoxycarbonyl steht, R₈ für Wasserstoff steht, Rⱼ für Wasserstoff oder Halogen steht und Rₖ für Cyano, Methoxycarbonyl, Aminocarbonyl, tert-Butoxycarbonyl, 2-Pyridyl-N-oxid, Nitro oder Vinyl steht.

10. Pharmazeutische Zusammensetzung, die eine Verbindung nach einem der Ansprüche 1 bis 9 oder ein pharmazeutisch annehmbares Salz hiervon und einen pharmazeutisch annehmbaren Träger umfasst.

11. Zusammensetzung nach Anspruch 10, die ferner ein β-Lactamantibiotikum umfasst.

12. Zusammensetzung nach Anspruch 10, worin das Antibiotikum Amoxicillin, Piperacillin, Ampicillin, Ceftizoxim, Cefotaxim, Cefuroxim, Cephalexin, Cefaclor, Cephaloridin oder Ceftazidime ist.

13. Verfahren, das die Hemmung einer β-Lactamase durch Zusammenbringen der β-Lactamase mit einer wirksamen Menge einer Verbindung nach einem der Ansprüche 1 bis 10 in vitro umfasst.

14. Verbindung nach einem der Ansprüche 1 bis 10 zur Verwendung in der medizinischen Therapie.

15. Verwendung einer Verbindung nach einem der Ansprüche 1 bis 10 zur Herstellung eines Arzneimittels zur Verringerung der β-Lactamaseaktivität bei einem Säuger.

16. Verbindung der Formel IV 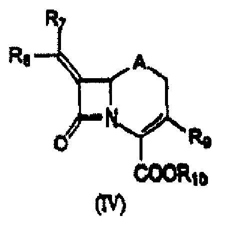 worin
R₇ und R₈ jeweils unabhängig für Wasserstoff, C₁-C₁₀ Alkyl, C₂-C₁₀ Alkenyl, C₂-C₁₀ Alkinyl, C₃-C₈ Cycloalkyl, C₁-C₁₀ Alkoxy, C₁-C₁₀ Alkanoyl, C₁-C₁₀ Alkanoyloxy, C₁-C₁₀ Alkoxycarbonyl, Aryl, einen Heterocyclus, Halogen, Cyano, Nitro, -COORₑ, -C(=O)NR_{f}R_{g}, -OC(=O)NR_{f}R_{g}, NR_{f}R_{g} oder -S(O)ₙRₕ stehen, R₉ für Cyano, -CH=NORᵢ oder einen Rest der folgenden Formel steht 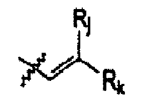 R₁₀ für C₁-C₁₀ Alkyl, C₂-C₁₀ Alkenyl, C₃-C₈ Cycloalkyl, C₂-C₁₀ Alkinyl, Aryl, Benzyl und Benzhydryl steht,
A für Thio, Sulfinyl oder Sulfonyl steht,
n jeweils unabhängig für 0, 1 oder 2 steht,
Rₑ jeweils unabhängig für Wasserstoff oder C₁-C₁₀ Alkyl steht,
R_{f} und R_{g} jeweils unabhängig für Wasserstoff, C₁-C₁₀ Alkyl, C₁-C₁₀ Alkoxy, Phenyl, Benzyl, Phenethyl oder C₁-C₁₀ Alkanoyl stehen,
Rₕ jeweils unabhängig für C₁-C₁₀ Alkyl, Phenyl, Aryl-C₁-C₆-alkyl, einen Heterocyclus oder Heterocyclus-C₁-C₆-alkyl steht,
Rᵢ für Wasserstoff oder C₁-C₆ Alkyl steht, und
Rⱼ und Rₖ jeweils unabhängig für Wasserstoff, Halogen, Cyano, Nitro, Aryl, einen Heterocyclus, C₂-C₆ Alkenyl, -COORₑ, -C(=O)NR_{f}Rg, -OC(=O)NR_{f}R_{g}, NR_{f}Rg oder -S(O)ₙRₕ stehen,
worin jeweils C₁-C₁₀ Alkyl, C₂-C₁₀ Alkenyl, C₂-C₁₀ Alkinyl, C₃-C₈ Cycloalkyl, C₁-C₁₀ Alkoxy, C₁-C₁₀ Alkanoyl, C₁-C₁₀ Alkanoyloxy oder C₁-C₁₀ Alkoxycarbonyl von R₇, R₈, Rⱼ und Rₖ optional mit einem oder mehreren (beispielsweise 1, 2, 3 oder 4) Substituenten substituiert sind, die unabhängig ausgewählt sind aus Halogen, Hydroxy, Cyano, Cyanato, Nitro, Mercapto, Oxo, Aryl, einen Heterocyclus, C₂-C₆ Alkenyl, C₂-C₆ Alkinyl, C₁-C₆ Alkoxy, C₁-C₆ Alkanoyl, C₁-C₆ Alkanoyloxy, Aryl-C₁-C₆-alkanoyloxy, Halogen-C₁-C₆-alkanoyloxy, Heterocyclus-C₁-C₆-alkanoyloxy, Aryloxy, (Heterocyclus)oxy, C₃-C₈ Cycloalkyl, -COORₑ, -C(=O)NR_{f}R_{g}, -OC(=O)NR_{f}R_{g}, NRₕRᵢ oder -S(O)ₙRₖ und
worin jedes Aryl wahlweise mit einem oder mehreren (beispielsweise 1, 2, 3 oder 4) Substituenten substituiert ist, die unabhängig ausgewählt sind aus Halogen, Hydroxy, Cyano, Trifluormethyl, Nitro, Trifluormethoxy, C₁-C₆ Alkyl, C₁-C₆ Alkanoyl, C₁-C₆ Alkanoyloxy, C₁-C₆ Alkoxycarbonyl, -COORₑ, -C(=O)NR_{f}R_{g}, -OC(=O)NR_{f}R_{g}, NRₕRᵢ oder -S(O)ₙRₖ.

## Revendications

1. Composé de formule IV : 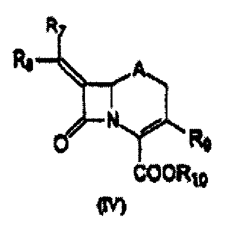 dans laquelle :
R₇ et R₈ représentent chacun indépendamment un atome d'hydrogène, un groupe (C₁-C₁₀) alkyle, (C₂-C₁₀) alcényle, (C₂-C₁₀) alcynyle, (C₃₋C₈) cycloalkyle, (C₁-C₁₀) alcoxy, (C₁-C₁₀) alcanoyle, (C₁-C₁₀) alcanoyloxy, (C₁₋C₁₀) alcoxycarbonyle, aryle, hétérocycle, halogéno, cyano, nitro, -COORₑ, - C(=O)NR_{f}R_{g}, -OC(=O)NR_{f}R_{g}, NR_{f}R_{g}, ou -S(O)ₙRₕ ;
R₉ représente un groupe cyano, -CH=NORᵢ, ou un radical de la formule suivante 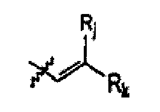
R₁₀ représente un atome d'hydrogène ;
A représente un groupe thio, sulfinyle, ou sulfonyle ;
chaque n a indépendamment la valeur de 0, 1, ou 2 ;
chaque Rₑ représente indépendamment un atome d'hydrogène, ou un groupe (C₁-C₁₀) alkyle;
chaque R_{f} et R_{g} représente indépendamment un atome d'hydrogène, un groupe (C₁-C₁₀)alkyle, (C₁-C₁₀)alcoxy, phényle, benzyle, phénéthyle, ou (C₁₋C₁₀)alcanoyle ;
chaque Rₕ représente indépendamment un groupe (C₁-C₁₀)alkyle, phényle, aryl(C₁-C₆)alkyle, hétérocycle, ou hétérocycle(C₁-C₆)alkyle;
R_{f} représente un atome d'hydrogène ou un groupe (C₁-C₆)alkyle; et
Rⱼ et Rₖ représentent chacun indépendamment un atome d'hydrogène, un groupe halogéno, cyano, nitro, aryle, hétérocycle, (C₂-C₆)alcényle, -COORₑ, -C(=O)NR_{f}R_{g}, -OC(=O)NR_{f}R_{g}, NR_{f}R_{g}, ou -S(O)ₙRₕ;
dans laquelle chaque groupe (C₁-C₁₀)alkyle, (C₂-C₁₀)alcényle, (C₂₋C₁₀)alcynyle, (C₃-C₈)cycloalkyle, (C₁-C₁₀)alcoxy, (C₁-C₁₀)alcanoyle, (C₁₋C₁₀)alcanoyloxy, ou (C₁-C₁₀)alcoxycarbanyle de R₇, R₈, Rⱼ et Rₖ est éventuellement substitué par un ou plusieurs (par exemple 1, 2, 3, ou 4) substituant(s) indépendamment choisi(s) parmi un groupe halogéno, hydroxy, cyano, cyanato, nitro, mercapto, oxo, aryle, hétérocycle, (C₂-C₆)alcényle, (C₂₋C₆)alcynyle, (C₁-C₆)alcoxy, (C₁-C₆)alcanoyle, (C₁-C₆)alcanoyloxy, aryl(C₁₋C₆)alcanoyloxy, halogéno(C₁-C₆)alcanoyloxy, hétérocycle(C₁-C₆)alcanoyloxy, aryloxy, (hétérocycle)oxy, (C₃-C₈)cycloalkyle, -COORₑ, -C(=O)NR_{f}R_{g}, -OC(=O)NR_{f}R_{g}, NRₕRᵢ, ou -S(O)ₙRₖ; et
dans laquelle n'importe quel groupe aryle est éventuellement substitué par un ou plusieurs (par exemple 1, 2, 3, ou 4) substituant(s) indépendamment choisi(s) parmi un groupe halogéno, hydroxy, cyano, trifluorométhyle, nitro, trifluorométhoxy, (C₁-C₆)alkyle, (C₁-C₆)alcanoyle, (C₁-C₆)alcanoyloxy, (C₁₋C₆)alcoxycarbonyle, -COORₑ, -C(=O)NR_{f}R_{g}, -OC(=O)NR_{f}R_{g}, NRₕRᵢ, ou -S(O)ₙRₖ ;
ou un sel pharmaceutiquement acceptable de celui-ci.

2. Composé selon la revendication 1, dans lequel R₇ représente un groupe aryle, hétérocycle, ou -COOR₈-.

3. Composé selon la revendication 1, dans lequel R₇ représente 2-pyridyle, ou -COOR₈-.

4. Composé selon l'une quelconque des revendications précédentes, dans lequel R₈ représente un atome d'hydrogène.

5. Composé selon l'une quelconque des revendications précédentes, dans lequel l'un parmi Rⱼ et Rₖ représente un atome d'hydrogène et l'autre représente un groupe cyano, -COOR₈, (C₂-C₁₀) alcényle, ou hétéroaryle.

6. Composé selon l'une quelconque des revendications 1 à 4, dans lequel Rⱼ représente un atome d'hydrogène ou un groupe halogéno, et Rₖ représente un groupe cyano, méthoxycarbonyle, aminocarbonyle, *tert*-butoxycarbonyle, 2-pyridyl-N-oxyde, nitro, ou vinyle.

7. Composé selon l'une quelconque des revendications précédentes, qui est un sel pharmaceutiquement acceptable d'un composé de formule IV dans laquelle R₁₁ représente un atome d'hydrogène.

8. Composé selon l'une quelconque des revendications précédentes, dans lequel A représente un groupe sulfonyle.

9. Composé selon la revendication 1, dans lequel A représente un groupe sulfonyle ; R₇ représente un groupe 2-pyridyle, carboxy ou *tert*-butoxy carbonyle ; R₈ représente un atome d'hydrogène ; Rⱼ représente un atome d'hydrogène ou un groupe halogéno ; et Rₖ représente un groupe cyano, méthoxycarbonyle, aminocarbonyle, *tert*-butoxycarbonyle, 2-pyridyl-N-oxyde, nitro, ou vinyle.

10. Composition pharmaceutique comprenant un composé selon l'une quelconque des revendications 1 à 9, ou un sel pharmaceutiquement acceptable de celui-ci; et un véhicule pharmaceutiquement acceptable.

11. Composition selon la revendication 10 comprenant en outre un antibiotique de type β-lactame.

12. Composition selon la revendication 10, dans laquelle l'antibiotique est l'amoxicilline, la pipéracilline, l'ampicilline, le ceftizoxime, le cefotaxime, le cefuroxime, la céphalexine, le céfaclor, la céphaloridine, ou le ceftazidime.

13. Procédé comprenant l'inhibition d'une β-lactamase par mise en contact de ladite β-lactamase avec une quantité efficace d'un composé selon l'une quelconque des revendications 1 à 10 *in vitro.*

14. Composé selon l'une quelconque des revendications 1 à 10 pour l'utilisation en thérapie médicale.

15. Utilisation d'un composé selon l'une quelconque des revendications 1 à 10 pour la fabrication d'un médicament de réduction de l'activité β-lactamase chez un mammifère.

16. Composé de formule IV 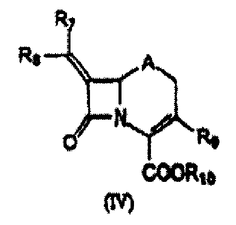 dans laquelle :
R₇ et R₈ représentent chacun indépendamment un atome d'hydrogène, un groupe (C₁-C₁₀)alkyle, (C₂-C₁₀)alcényle, (C₂-C₁₀)alcynyle, (C₃-C₈)cycloalkyle, (C₁-C₁₀)alcoxy, (C₁-C₁₀)alcanoyle, (C₁-C₁₀)alcanoyloxy, (C₁₋C₁₀)alcoxycarbonyle, aryle, hétérocycle, halogéno, cyano, nitro, -COORₑ, - C(=O)NR_{f}R_{g}, -OC(=O)NR_{f}R_{g}, NR_{f}R_{g}, ou -S(O)ₙRₕ ;
R₉ représente un groupe cyano, -CH=NORᵢ, ou un radical de formule suivante 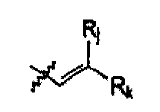
R₁₀ représente un groupe (C₁-C₁₀)alkyle, (C₂-C₁₀)alcényle, (C₃₋C₈)cycloalkyle, (C₂-C₁₀)alcynyle, aryle, benzyle, et benzhydryle ;
A représente un groupe thio, sulfinyle, ou sulfonyle ;
chaque n a indépendamment la valeur de 0, 1, ou 2 ;
chaque Rₑ représente indépendamment un atome d'hydrogène, ou un groupe (C₁-C₁₀)alkyle;
chaque R_{f} et R_{g} représente indépendamment un atome d'hydrogène, un groupe (C₁-C₁₀)alkyle, (C₁-C₁₀)alcoxy, phényle, benzyle, phénéthyle, ou (C₁₋C₁₀)alcanoyle;
chaque Rₕ représente indépendamment un groupe (C₁-C₁₀)alkyle, phényle, aryl(C₁-C₆)alkyle, hétérocycle, ou hétérocycle(C₁-C₆) alkyle;
Rᵢ représente un atome d'hydrogène ou un groupe (C₁-C₆) alkyle ; et
Rⱼ et Rₖ représentent chacun indépendamment un atome d'hydrogène, un groupe halogéno, cyano, nitro, aryle, hétérocycle, (C₂-C₆)alcényle, -COOR_{e,} -C(=O)NR_{f}R_{g}, -OC(=O)NR_{f}R_{g}, NR_{f}R_{g}, ou -S(O)RₙRₕ;
dans laquelle chaque groupe (C₁-C₁₀)alkyle, (C₂-C₁₀)alcényle, (C₂₋C₁₀)alcynyle, (C₃-C₈)cycloalkyle, (C₁-C₁₀)alcoxy, (C₁-C₁₀)alcanoyle, (C₁₋C₁₀)alcanoyloxy, ou (C₁-C₁₀)alcoxycarbonyle de R₇, R₈, Rⱼ et Rₖ est éventuellement substitué par un ou plusieurs (par exemple 1, 2, 3, ou 4) substituant(s) indépendamment choisi(s) parmi un groupe halogéno, hydroxy, cyano, cyanato, nitro, mercapto, oxo, aryle, hétérocycle, (C₂-C₆)alcényle, (C₂₋C₆)alcynyle, (C₁-C₆)alcoxy, (C₁-C₆)alcanoyle, (C₁-C₆)alcanoyloxy, aryl(C₁₋C₆)alcanoyloxy, halogéno(C₁-C₆)alcanoyloxy, hétérocycle(C₁-C₆)alcanoyloxy, aryloxy, (hétérocycle)oxy, (C₃-C₈)cycloalkyle, -COORₑ, -C(=O)NR_{f}Rg, -OC(=O)NR_{f}R_{g}, NRₕRᵢ, ou -S(O)ₙRₖ ; et
dans laquelle n'importe quel groupe aryle est éventuellement substitué par un ou plusieurs (par exemple 1, 2, 3, ou 4) substituant(s) indépendamment choisi(s) parmi un groupe halogéno, hydroxy, cyano, trifluorométhyle, nitro, trifluorométhoxy, (C₁-C₆) alkyle, (C₁-C₆) alcanoyle, (C₁-C₆) alcanoyloxy, (C₁₋C₆) alcoxycarbonyle, -COORₑ, -C(=O)NR_{f}R_{g}, -OC(=O)NR_{f}R_{g}, NRₕRᵢ, ou -S(O)ₙRₖ.
